Europäisches Patentamt

European Patent Office

Office européen des brevets

(11) Veröffentlichungsnummer: **0 384 458 B1**

(19)

(12) # EUROPÄISCHE PATENTSCHRIFT

(45) Veröffentlichungstag der Patentschrift: **22.12.93**

(21) Anmeldenummer: **90103447.0**

(22) Anmeldetag: **22.02.90**

(51) Int. Cl.5: **B01D 3/00**, B01D 3/40,
B01D 3/34, B01D 3/36,
C07C 45/83

(54) ## Verfahren zur Trennung von diacetylhaltigen Gemischen aus Methylethylketon, Ethylacetat, Ethanol, Wasser sowie gegebenenfalls Toluol und n-Hexan.

(30) Priorität: **24.02.89 DE 3905786**

(43) Veröffentlichungstag der Anmeldung:
**29.08.90 Patentblatt 90/35**

(45) Bekanntmachung des Hinweises auf die
Patenterteilung:
**22.12.93 Patentblatt 93/51**

(84) Benannte Vertragsstaaten:
**AT BE CH ES FR GB IT LI NL**

(56) Entgegenhaltungen:
**US-A- 2 702 783**
**US-A- 2 862 853**
**US-A- 4 470 881**

**DERWENT ACCESSION, no. 88-041178(06),**
**Ouestel Telesystems (WPIL), Derwent Publi-**
**cations Ltd., London (GB)&NUM;**

**DERWENT ACCESSION, no. 70-37442R(21),**
**Ouestel Telesystems (WPIL), Derwent Publi-**
**cations Ltd., London (GB)&NUM;**

(73) Patentinhaber: **Wipf AG Verpackungen**
**Industriestrasse**
**CH-8604 Volketswil(CH)**

(72) Erfinder: **Simmrock, Karl-Hans,**
**Prof.Dipl.-Chem.Dr.**
**Karoline Zorwald-Strasse 4**
**D-4600 Dortmund(DE)**
Erfinder: **Schembecker, Gerhard, Dipl.-Ing.**
**Ackfelder Strasse 4**
**D-4724 Wadersloh(DE)**
Erfinder: **Cyris, Peter**
**Blaumenacker 17**
**D-4600 Dortmund 50(DE)**

(74) Vertreter: **Betten & Resch**
**Reichenbachstrasse 19**
**D-80469 München (DE)**

EP 0 384 458 B1

**Beschreibung**

Die Erfindung betrifft ein Verfahren zur Trennung von diacetylhaltigen Gemischen aus Methylethylketon, Ethylacetat, Ethanol und Wasser, die gegebenenfalls Toluol, Isopropanol, Essigsäure und Kohlenwasserstoffe, insbesondere n-Hexan, enthalten, wie sie insbesondere in der Druckindustrie als Abfallprodukte anfallen, zur Rückgewinnung von wiederverwendbarem Methylethylketon (mit etwa 20 Gew.-% Ethylacetat, höchstens etwa 0,5 Gew.-% Ethanol und höchstens etwa 0,1 Gew.-% Wasser), Ethylacetat (mit höchstens etwa 1 Gew.-% Methylethylketon, höchstens etwa 0,5 Gew.-% Ethanol und höchstens etwa 0,05 Gew.-% Wasser) und Ethanol (mit höchstens etwa 0,1 Gew.-% Methylethylketon, höchstens etwa 1 Gew.-% Wasser und höchstens etwa 1 Gew.-% Ethylacetat) von Lösungsmittelqualität.

Die umweltfreundliche und wirtschaftliche Aufarbeitung von Abfall-Lösungsmittelgemischen, wie sie in der chemischen Industrie, insbesondere in der Druckindustrie, in großen Mengen anfallen, ist ein Problem, das immer mehr an Bedeutung gewinnt, da die einschlägigen gesetzlichen Vorschriften zunehmend strenger werden, so daß die zulässigen Höchstgrenzen für Lösungsmittelgehalte in der Abluft und in Abwässern immer weiter herabgesetzt werden, um vermutete oder tatsächlich aufgetretene Umweltschäden zu verhindern.

Man ist daher seit langem bemüht, derartige Abfall-Lösungsmittelgemische möglichst wirtschaftlich so wiederaufzuarbeiten, daß die darin enthaltenen Lösungsmittelkomponenten in geeigneten chemischen Prozessen wiederverwendet werden können.

Typische, hauptsächlich in der Druckindustrie als Abfallprodukte anfallende Lösungsmittelgemische bestehen aus

etwa 25 bis etwa 30 Gew.-% Methylethylketon (MEK)

etwa 25 bis etwa 35 Gew.-% Ethylacetat (EtOAc)

etwa 20 bis etwa 25 Gew.-% Ethanol (EtOH)

etwa 10 bis etwa 15 Gew.-% Wasser ($H_2O$)

etwa 1 Gew.-% Isopropanol (IPA)

etwa 0,5 Gew.-% Toluol (Tol)

etwa 0,1 Gew.-% Kohlenwasserstoffe, vorzugsweise n-hexan (Hex)

etwa 2,5 Gew.-% Ethoxypropanol + Propylenglycolmethyläther (Etoxy),

etwa 0,2 Gew.-% Essigsäure (AcOH) und

etwa 0,2 Gew.-% Diacetyl (Diac),

aus denen Methylethylketon, Ethanol und Ethylacetat frei von Diacetyl zurückgewonnen werden sollen, so daß sie als Lösungsmittel wiederverwendbar sind. Um diese Bedingungen zu erfüllen, müssen sie folgenden Reinheitskriterien genügen:

Methylethylketon darf höchstens etwa 20 Gew.-% Ethylacetat, höchstens etwa 0,5 Gew.-% Ethanol und höchstens etwa 0,1 Gew.-% Wasser enthalten,

Ethylacetat darf höchstens etwa 1 Gew.-% Methylethylketon, höchstens etwa 0,5 Gew.-% Ethanol und höchstens etwa 0,05 Gew.-% Wasser enthalten und

Ethanol darf höchstens etwa 1 Gew.-% Methylethylketon, höchstens etwa 1 Gew.-% Wasser und höchstens etwa 1 Gew.-% Ethylacetat enthalten.

Das darin enthaltene Diacetyl ist wegen seines Geruches und seiner intensiven Färbung so weitgehend zu entfernen, daß die zurückgewonnenen Lösungsmittel einer sensorischen Prüfung standhalten.

Es gibt zwar bereits zahlreiche Verfahren zur Trennung von Mehrstoffgemischen, auch solchen der vorgenannten Art, diese sind jedoch entweder technisch außerordentlich aufwendig und daher unwirtschaftlich, oder sie liefern die gewünschten Regenerate nicht in der erforderlichen Qualität.

So ist beispielsweise aus der US-PS 2 702 783 ein Verfahren zur Auftrennung eines Ausgangsgemisches aus Ethylacetat, Ethanol, Methylethylketon und Wasser bekannt, das die Abtrennung eines Ethylacetat/Ethanol/$H_2O$-Azeotrops als Kopfprodukt einer Fraktionierkolonne und die Extraktion von Ethanol aus dem Kopfprodukt mit Wasser als Lösungsmittel, die Reindarstellung von Ethylacetat aus dem dabei erhaltenen Raffinat als Sumpfprodukt einer weiteren Ethylacetat/$H_2O$-Azeotrop-Destillation und die Abtrennung von Ethylacetat aus dem bei der Extraktion erhaltenen Extrakt als azeotropes Gemisch mit Ethanol und Wasser umfaßt.

Aus der US-PS 2 862 853 ist die Zurückgewinnung von Methylethylketon aus einem Gemisch mit 30 bis 60 Gew.-% Methylethylketon, 5 bis 7 Gew.-% niederen aliphatischen Alkoholen (z.B. Isopropanol, Ethanol), 10 bis 70 Gew.-% Estern von niederen aliphatischen Alkoholen (z.B. Ethylacetat) und Wasser bekannt. Bei diesem bekannten Verfahren erfolgt die Trennung in zwei Extraktiv-Destillationskolonnen, die jeweils mit Wasser betrieben werden. Als Kopfprodukt wird dabei eine Methylethylketon-Ester-Fraktion erhalten, während als Sumpfprodukt eine Alkohol-Wasser-Fraktion erhalten wird. Das Kopfprodukt wird einer

EP 0 384 458 B1

weiteren Extraktiv-Destillation unter Zulauf von Wasser als Lösungsmittel unterzogen.

Es sind auch bereits zahlreiche weitere Verfahren zur Trennung von Mehrstoffsystemen bekannt, in denen die unterschiedlichsten Lösungsmittel und/oder Extraktionsmittel, vorzugsweise Diethylenglycol und Dimethylformamid, zur Durchführung von Fraktionierungen und/oder Extraktionen eingesetzt werden.

Allen diesen Verfahren zur Auftrennung von Stoffgemischen ist jedoch gemeinsam, daß sie Probleme mit sich bringen, wenn in dem Ausgangsgemisch Diacetyl und Isopropanol als Verunreinigungen enthalten sind.

Aufgabe der Erfindung war es daher, eine Möglichkeit zu finden, mit deren Hilfe es möglich ist, auf technisch einfache und wirtschaftliche Weise Lösungsmittelgemische der eingangs genannten Art, wie sie insbesondere in der Druckindustrie als Abfallprodukte anfallen, so aufzubereiten, daß die darin enthaltenen Hauptkomponenten Methylethylketon, Ethanol und Ethylacetat als Lösungsmittel wiederverwendbar sind und die übrigen darin enthaltenen Komponenten in einer Form erhalten werden, die ihre umweltunschädliche Beseitigung ermöglicht.

Es wurde nun gefunden, daß diese Aufgabe erfindungsgemäß dadurch gelöst werden kann, daß ein Lösungsmittel-Ausgangsgemisch der vorgenannten Art einer Extraktiv-Rektifikation unter Verwendung von Wasser als Hilfsstoff und anschließend einer Gegenstom-Extraktion unter Rückführung des Extraktes, in der ebenfalls Wasser als Hilfsmittel verwendet wird, unterzogen wird.

Gegenstand der Erfindung sind Verfahren zur Trennung von diacetylhaltigen Gemischen der eingangs genannten Art, wie sie in den beiliegenden Patentansprüchen charakterisiert sind.

Das den Gegenstand der Erfindung bildende Verfahren gemäß den beiliegenden Patentansprüchen bietet gegenüber den Verfahren gemäß Stand der Technik die folgenden technischen Vorteile:

(a) Die Verwendung von zusätzlichen, nicht im Ausgangsgemisch vorhandenen fremden Hilfsstoffen wird vermieden;

(b) das Verfahren stabilisiert sich in seinen Kreisläufen weitgehend selbst, falls an einer Stelle im Verfahren die vorgesehenen Reinheiten oder Mengenströme nicht erfüllt werden; und

(c) sowohl die Abtrennung des Diacetyls von Methylethylketon und Ethylacetat als auch die weitere Auftrennung der dabei erhaltenen Massenströme werden weitestgehend mit dem umweltfreundlichen Rektifikations-Hilfsmittel bzw. Lösungsmittel Wasser durchgeführt.

Der obengenannte Vorteil (b) ist insbesondere dann besonders ausgeprägt, wenn beim Aufarbeiten von aus mehreren Quellen stammenden Lösungsmittelgemischen weder die

Zusammensetzungen noch die durchzusetzenden Mengen der einzelnen Komponenten genau bekannt sind.

Die Verwendung von Wasser als Hilfsstoff in der Extraktiv-Rektifikations-Stufe bietet darüber hinaus die folgenden Vorteile:

- EtOH, Etoxy und IPA werden in den Sumpf der Extraktiv-Rektifikations-Kolonne gedrückt,
- MEK und EtOAc gehen nahezu quantitativ in das Destillat,
- das Diacetyl kann im Sumpfablauf der ersten Stufe durch Alkalizusatz zerstört werden,
- das Destillat ist arm an Wasser (Azeotropbildung),
- für das anfallende Destillat, bestehend aus MEK, EtOAc und $H_2O$, sind wirtschaftliche Aufarbeitungsmöglichkeiten gegeben,
- der Siedepunkt des Wassers liegt niedrig, so daß es leicht gestrippt werden kann,
- die Regeneration des Hilfsstoffes ist, zumindest aus Kostengründen, nicht notwendig und
- die Aufkonzentrierung der im Sumpf enthaltenen Alkohole EtOH und IPA ist leicht möglich.

Das einen Gegenstand der Erfindung bildende erfindungsgemäße Verfahren zur Trennung von diacetylhaltigen Gemischen der vorgenannten Art zeichnet sich insbesondere durch die nachstehend angegebene Kombination von Verfahrensmaßnahmen aus, wobei darauf hinzuweisen ist, daß auch die einzelnen Verfahrensmaßnahmen für sich allein erfinderisch sind, insbesondere

($\alpha$) die Abtrennung von Diacetyl aus dem diacetylhaltigen Ausgangsgemisch,

($\beta$) die Gegenstrom-Extraktion mit Rückführung der organischen Phase des ternären Gemisches aus Methylethylketon, Ethylacetat und Wasser zur Erzeugung von Methylethylketon/Ethylacetat in einem Gewichtsverhältnis von (50 bis 85) : (15 bis 50), vorzugsweise 80 : 20, sowie von wasserfreiem Ethylacetat und

($\gamma$) die azeotrope Entwässerung von Methylethylketon/Ethylacetat und von Ethylacetat mittels Kohlenwasserstoffen und Äthern, vorzugsweise Äthern, insbesondere Methyl-tert-butyläther:

a) die in dem Ausgangsgemisch gegebenenfalls enthaltene Essigsäure wird durch Neutralisation mit Natriumcarbonat eliminiert;

b) das durch Farbe und Geruch störende, in dem neutralisierten Ausgangsgemisch enthaltene Diacetyl wird durch Extraktiv-Rektifikation mit Wasser und nachfolgende Umsetzung mit Alkali- oder

3

Erdalkalihydroxiden, Alkali- oder Erdalkalicarbonaten oder Oximen entfernt oder als unterer Seitenstrom in aufkonzentrierter Form kurz oberhalb des bei der Extraktiv-Rektifikation entstehenden Sumpfes abgezogen;

c) das bei der Extraktiv-Rektifikation in der Stufe (b) entstehende Destillat wird als oberer Seitenstrom abgezogen und durch Gegenstrom-Extraktion mit Extraktrückführung in ein Mischlösungsmittel aus etwa 80 Gew.-% Methylethylketon und etwa 20 Gew.-% Ethylacetat (wasserfrei gerechnet) und in nahezu wasserfreies Ethylacetat zerlegt;

d) die Entfernung des restlichen Wassers aus dem bei der Gegenstrom-Extraktion in der Stufe (c) entstehenden Raffinat erfolgt durch azeotrope Entwässerung mittels Äthern oder Kohlenwasserstoffen, deren azeotrope Siedepunkte mit Wasser unterhalb 70 °C liegen;

e) Auftrennung des in der Stufe (d) erhaltenen Sumpfprodukts durch Rektifikation in praktisch reines Ethylacetat und praktisch reines Toluol;

f) Rückgewinnung und Rückführung der Hauptmenge des als Lösungsmittel für die Gegenstrom-Extraktion dienenden Wassers aus dem Extrakt der Gegenstrom-Extraktion durch Abdestillieren und Rückführung eines Teils der organischen Phase des dabei entstehenden kondensierten ternären Methylethylketon-Ethylacetat-Wasser-Gemisches in die Gegenstrom-Extraktion; und

g) Entfernung des restlichen Wassers aus dem ternären Methylethylketon-Ethylacetat-Wasser-Gemisches durch azeotrope Entwässerung mittels Äthern oder Kohlenwasserstoffen, deren azeotrope Siedepunkte mit Wasser unterhalb 70 °C liegen, unter Bildung eines Sumpfprodukts aus Methylethylketon und Ethylacetat im Gewichtsverhältnis 80:20.

Die Erfindung wird nachstehend unter Bezugnahme auf die beiliegenden Zeichnungen näher erläutert, wobei zeigen:

Abb. 1 ein Fließbild, das die Durchführung des erfindungsgemäßen Verfahrens in schematischer Darstellung erläutert;

Abb. 2 ein Fließbild, das die Durchführung des erfindungsgemäßen Verfahrens in einer praxisnahen Ausführungsform mit zusätzlich eingezeichneten Wärmetauschern W (Kondensatoren, Verdampfern, Vorwärmern), Behältern B und Abscheidern A, erläutert; und

Abb. 3 ein Fließbild des erfindungsgemäßen Verfahrens, aus dem die qualitativen und quantitativen Zusammensetzungen der Massenströme bei Durchführung des erfindungsgemäßen Verfahrens ersichtlich sind.

Das erfindungsgemäße Verfahren wird nachstehend anhand einer detaillierten Beschreibung der einzelnen Verfahrensschritte näher erläutert.

Es bedeuten:

S(xx)      : Massenstrom xx
W(xx)      : Wärmetauscher xx (Kondensator, Verdampfer, Vorwärmer)
B(xx)      : Behalter xx
K(x)       : Rektifikationskolonne Nr.(x)
Feed       : zu regenerierendes Ausgangsgemisch S(1)

Konzentrationen und Massenströme sind in den nachstehenden Schaubildern aufgeführt.

Bezeichnung der Stoffe:

Diac       : Diacetyl
EtOAc      : Ethylacetat
EtOH       : Ethanol
Ethoxy     : Ethoxypropanol + Propylenglykolmethylether
MEK        : Methylethylketon
MTBE       : Methyl-tert.-butylether
Tol        : Toluol
IPA        : Isopropanol

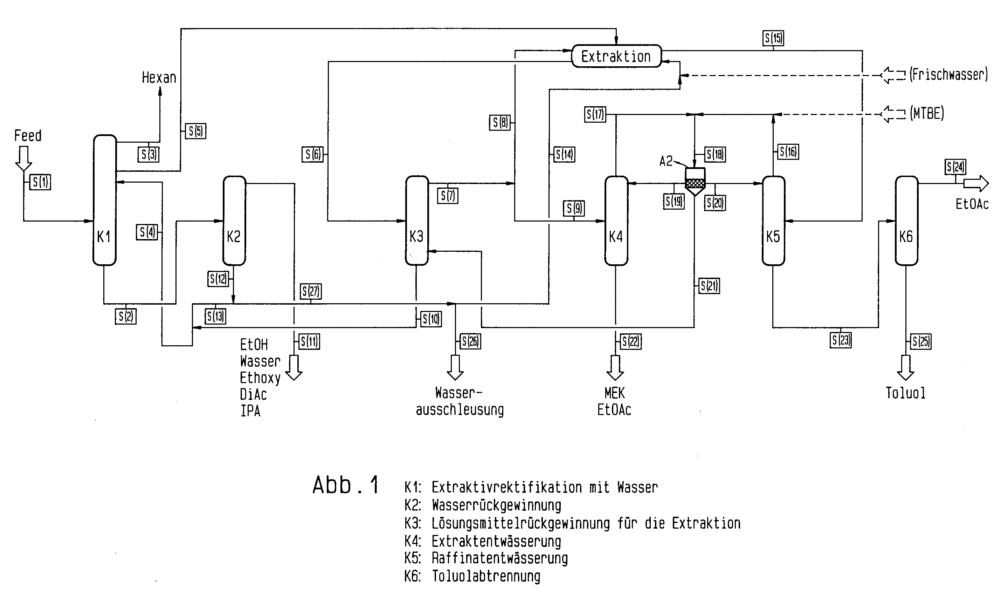

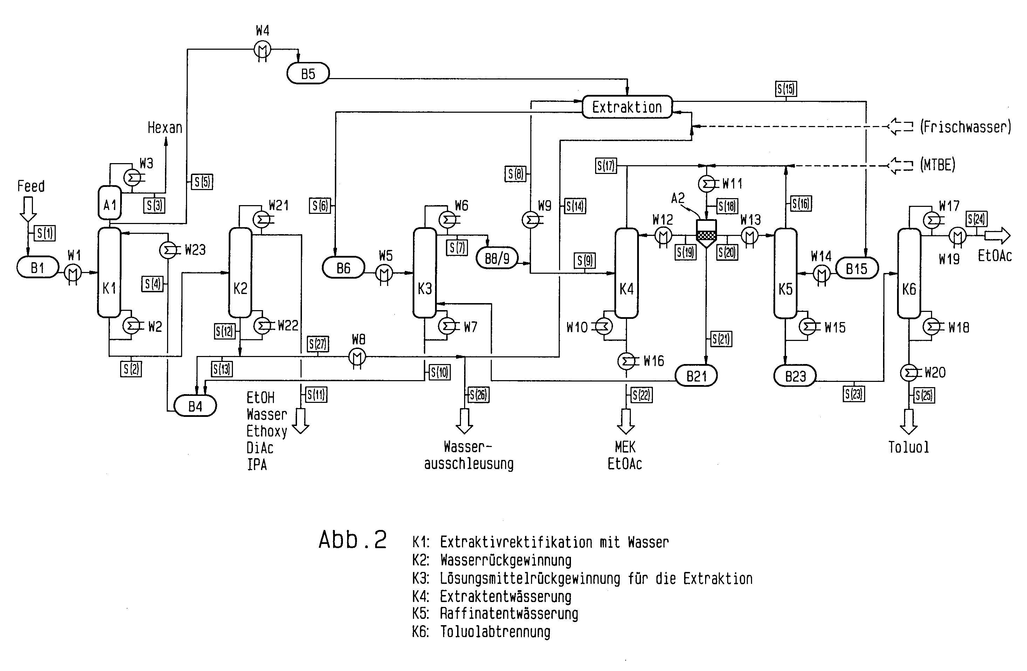

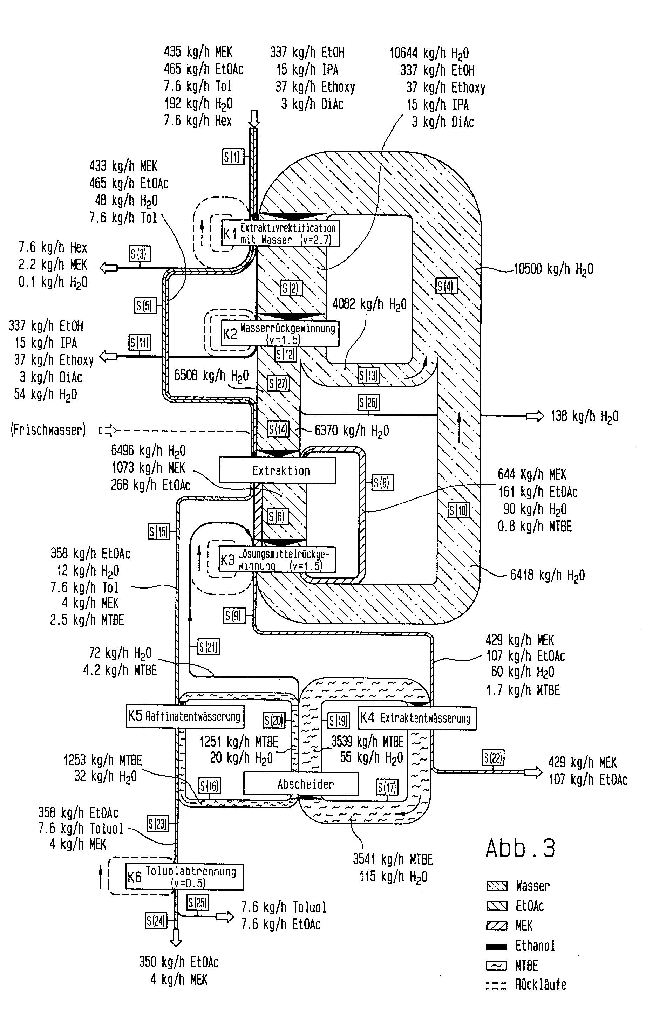

Das Fließbild des erfindungsgemäßen Verfahrens ist in Abb. 1 und Abb. 2 dargestellt. Die qualitative und quantitative Zusammensetzung der in den einzelnen Stufen fließenden Massenströme ist in Abb. 3 dargestellt.

In der Rektifikationskolonne K(1) wird durch Extraktivrektifikation mit einem zurückgeführten Wasserstrom S(4) der aus dem Lagerbehälter B(1) kommende neutralisierte Zulauf S(1) in die drei Ströme S(2), S-(3) und S(5) zerlegt. (Abb. 2).

Am Kopf der Kolonne K(1) wird ein Gemisch S(3) aus ca. 1/4 MEK und 3/4 n-Hexan (mit ca. 1 % Wasser) als obere Phase im PhasenabscheiderA(1)(Abb.2) abgezogen; die untere, wäßrige Phase fließt in die Kolonne zurück (Abb. 2). Das anschließend getrocknete Lösungsmittelgemisch aus n-Hexan und MEK wird der weiteren Verwendung zugeführt.

Mit dem Sumpf S(2) wird das gesamte Diacetyl zusammen mit EtOH, IPA, Etoxy (sowie evtl. vorhandenen geringen Mengen Natriumacetat aus der vorgeschalteten Essigsäureneutralisation) in wäßriger Lösung abgezogen. Eine weitgehende Entfernung von MEK und EtOAc aus dem Sumpfablauf ist zweckmäßig, da diese Komponenten des Zulaufs S(1) ansonsten bei einer möglichen Rückgewinnung der Alkohole aus dem Abwasser in der dafür erforderlichen Kolonne K(2) gemeinsam mit EtOH/IPA/-Etoxy im Destillat S-(11) erscheinen.

Darüber hinaus hat sich bei den Versuchen gezeigt, daß Diacetyl kurz oberhalb des Sumpfes angereichert ist und bequem als unterer Seitenstrom in aufkonzentrierter Form abgezogen werden kann.

Als oberer Seitenstrom wird aus der Kolonne K(1) ein Destillat S(5) abgezogen, das - frei von Diacetyl und auch frei von Alkoholen - etwa je zur Hälfte aus MEK und EtOAc, neben 5 % Wasser und ca. 1 % Toluol, besteht. Diesen Strom S(5) weitgehend frei von EtOH zü halten ist ein günstiger Nebeneffekt, da EtOH, MTBE und Wasser ein ternäres Azeotrop bilden, was sich bei der späteren Entwässerung in den Kolonnen K(4) und K(5) nachteilig auswirken würde (siehe dort).

Das als Rektifikationshilfsstoff eingesetzte Wasser S(4) ist ein Regenerat (Teil des Sumpfabzugs) aus den Entwässerungs-Kolonnen K(2) und K(3). Geringe Restgehalte an MEK- und EtAOc stören nicht.

In der Kolonne K(2)wird aus dem Sumpfprodukt S(2) der Kolonne K(1) der Hilfsstoff Wasser für die Kreisläufe zurückgewonnen, indem man ein Gemisch aus EtOH/IPA/Etoxy/Diacetyl/Wasser als Destillat S-(11) abzieht. Der Sumpfstrom S(12) dient zur Extraktiv-Rektifikation in Kolonne K(1) und als Lösungsmittel S(27) bzw. S(14) der Extraktion.

Eine dem Wassergehalt des Feed und der Frischwasserzufuhr zum Lösungsmittel S(14) entsprechende Menge S(26) des Stromes S(27) muß hier ausgeschleust werden. Mit dieser Ausschleusung verläßt auch das in geringen Mengen bei der Neutralisation gebildete Natriumacetat das Verfahren, falls es nicht schon zuvor aus dem Zulauf der Kolonne K(1) entfernt worden war.

Das Destillat S(11) kann nach einem der bekannten Verfahren der Alkoholabsolutierung vom Wasser befreit werden. In dieser, im Fließbild nicht aufgeführten Einrichtung, wird das Diacetyl durch Zugabe kleiner Mengen Alkalihydroxid oder Alkalicarbonat durch Kochen zerstört bzw. aufwendiger durch Reagentien wie Hydroxylamin, Alkalisulfit oder dgl. chemisch gebunden. Auf diese Weise läßt sich Diacetyl, ohne Verluste durch Verseifung von EtOAc oder durch Umsetzung von MEK, die beide bereits aus dem Gemisch entfernt wurden, dem Gemisch entziehen. Das dort mit tiefschwarzen Zersetzungsprodukten des Diacetyls in sehr geringer Menge als Sumpfprodukt anfallende Abwasser muß ausgeschleust werden.

Das Destillat S(5) wird zu der mit Wasser S(14) als Lösungsmittel ausgeführten Gegenstromextraktion (mit Extraktrücklauf S(8)) geführt.

In der Gegenstromextraktion mit Extraktrücklauf fällt als Extrakt eine wäßrige Lösung S(6) an, in der das Verhältnis MEK:EtOAc mindestens 4:1 beträgt. Dieser Extrakt S(6) enthält ungefähr 83 % Wasser.

Im Raffinat S(15) befindet sich neben ca. 93 % EtOAc und 3 % Wasser praktisch das gesamte Toluol (ca. 2 %). Die Menge des Extraktionsmittels Wasser und der Extraktrücklauf sind so aufeinander abzustimmen, daß der MEK-Gehalt des Raffinatgehaltes 1 Gew% nicht überschreitet, um die Reinheitsanforderung des EtOAc zu erfüllen.

Die Aufarbeitung des Extraktstromes S(6) beginnt mit der Vor-Entwässerungs-Kolonne K(3).

Dort erhält man ein Destillat S(7), bestehend aus einem wassergesättigten Gemisch von 72 % MEK und 18 % EtOAc, das teils als Extraktrücklauf S(8) dient und teils zur Befreiung von restlichem Wasser der Kolonne K(4) als S(9) zugeführt wird.

Im Sumpf fällt Wasser S(10) an, das zur Extraktiv-Rektifikation in Kolonne K(1) dient. Durch diese Rückführung verhält sich die Kolonne K(3) betriebsstabil gegen Restkonzentrationen im Sumpfstrom S(10). So kann auf diesem Wege sichergestellt werden, daß eventuell in den Extraktzulauf S(5) in geringen Mengen geratenes EtOH, das letztlich bei der Entwässerung mit MTBE als ternäres Azeotrop MTBE/Wasser/EtOH im Strom S(18) erscheint, über die wäßrige Phase S(21) des dortigen Abscheiders und über den Sumpfstrom S(10) aus Kolonne K(3) wieder in die Hauptkolonne K(1) zurückströmt. Von dort gelangt es mit dem bereits vorhandenen EtOH in den Sumpfablauf S(2). Diese Schaltung führt zu einer Selbststabilisierung des Verfahrens, die sich aus den obengenannten Gründen als vorteilhaft herausgestellt hat.

In der Kolonne K(4) wird das ca. 10 % Wasser enthaltende MEK/EtOAc-Gemisch durch Azeotrop-Rektifikation mit beispielsweise MTBE entwässert. Das im Verhältnis 4:1 anfallende, wasserfreie Mischlösungsmittel S(22) aus MEK und EtOAc wird dampfförmig aus dem Sumpf abgezogen, um ein sauberes Produkt zu gewährleisten.

Die aus der Extraktion stammende, vornehmlich EtOAc enthaltende, wassergesättigte Raffinatphase S-(15) wird in Kolonne K(5) ebenfalls durch Azeotrop-Destillation mit beispielsweise MTBE entwässert.

Die Destillate S(17) und S(16) aus den Kolonnen K(4) und K(5) sind beide Azeotrope von MTBE mit Wasser und können demzufolge gemeinsam als Strom S(18) auf einen gemeinsamen AbscheiderA(2)-geführt werden.

Der wasserfreie Sumpfabzug S(23) aus Kolonne K(5) enthält ca. 97 % EtOAc, 2 % Toluol und 1 % MEK und kann durch einfache kontinuierliche oder diskontinuierliche Rektifikation in Kolonne K(6) in ein Toluol S-(25) mit ca.1 %Rest-EtOAc und ein Destillat S(24) mit 99 % EtOAc und 1 % MEK zerlegt werden.

Die Erfindung wird in den folgenden Beispielen näher erläutert, ohne darauf beschränkt zu sein.

Ausführungsbeispiele

1) Die einfache Rektifikation eines Gemisches aus

| | |
|---|---|
| Methylethylketon | 36 Gew% |
| Ethanol | 23 Gew% |
| Ethylacetat | 30 Gew% |
| Wasser | 11 Gew% |
| Toluol | Spuren |
| Diacetyl | 0.2 Gew% |

wurde in einem viertägigen Dauerversuch diskontinuierlich in einer 60-bödigen Kolonne bei sehr hohem Rücklauf fraktioniert und sorgfältig analysiert.

Trotz hoher Bodenzahl und hohem Rücklaufverhältnis konnte in dieser Rektifikation ohne Zusatz weiterer Hilfsstoffe nur eine Trennung in die Fraktionen

| 1.Schnitt: | 60 Mol% Ethylacetat<br>30 Mol% Wasser<br>6 Mol% Methylethylketon<br>4 Mol% Ethanol | Siedetemperatur ca. 70 °C (Kp) |
|---|---|---|
| 2.Schnitt: | 55 Mol% Methylethylketon<br>27 Mol% Wasser<br>18 Mol% Ethanol | Siedetemperatur ca. 73 °C (Kp) |
| 3.Schnitt | 90 Mol% Ethanol<br>10 Mol% Wasser | Siedetemperatur ca. 78 °C (Kp) |

erreicht werden, wobei das Diacetyl über alle Fraktionen verteilt war, wie sich an der Gelbfärbung feststellen ließ. Keiner der vorhandenen Stoffe konnte, weder alleine noch als Gemisch, in gewünschter Reinheit erhalten werden. Dieses Verhalten ist auf das Auftreten von Azeotropen zwischen den eng siedenden Komponenten zurückzuführen.

Folgende Azeotrope sind aus der Literatur bekannt:

| Ternäre Azeotrope | | | | |
|---|---|---|---|---|
| Kp. | Ethylacetat | Ethanol | Wasser | Methylethylketon |
| 70,2° | 58 Mol%<br>83 Gew% | 11 Mol%<br>8 Gew% | 31 Mol%<br>9 Gew% | |
| 72,0° | 40 Mol %<br>54 Gew% | 50 Mol%<br>35 Gew% | | 10 Mol%<br>11 Gew% |

| Binäre Azeotrope: (Literaturwerte) | | | | | |
|---|---|---|---|---|---|
| | Kp. | Ethylacetat | Ethanol | Methylethylketon | Wasser |
| Ethylacetat | 77.1 ° | | 72,2 °<br>40 Mol%<br>26 Gew% | 76,7 °<br>15 Mol%<br>13 Gew% | 70,5 °<br>30 Mol%<br>8 Gew% |
| Ethanol | 78,3 ° | 72,2 °<br>60 Mol%<br>74 Gew% | | 74,3 °<br>50 Mol%<br>61 Gew% | 78,2 °<br>10 Mol%<br>4 Gew% |
| Methylethylketon | 79,9 ° | 76,7 °<br>85 Mol%<br>87 Gew% | 74,3 °<br>50 Mol%<br>39 Gew% | | 73,3 °<br>35 Mol%<br>88 Gew% |
| Wasser | 100 ° | 70,5 °<br>70 Mol%<br>92 Gew% | 78,2 °<br>90 Mol%<br>96 Gew% | 73,3 °<br>65 Mol%<br>88Gew% | |

Dieser Versuch zeigt, daß die einfache Rektifikation selbst unter sehr hohem Rücklauf mit sehr wirkungsvollen Kolonnen nicht zum gewünschten Erfolg führen kann.

2) Abtrennung des Diacetyls von MEK und EtOAc

Die direkte Zerstörung des Diacetyls in einer wäßrigen Lösung in Gegenwart von MEK und EtOAc durch längeres Erhitzen mit einem Alkalihydroxid, Alkalicarbonat, Erdalkalicarbonat verbietet sich, da sich Alkalien, wie die ausgeführten Versuche zeigen, vornehmlich mit dem Ester unter Bildung von Alkohol und Alkaliacetat umsetzen, es also zu Verlusten an EtOAc, verbunden mit zusätzlicher Abwasserbelastung, kommt. Eine Umsetzung mit carbonylspezifischen Reagentien in solchen Lösungen entfällt ebenfalls, da diese vornehmlich mit dem in hoher Konzentration vorhandenen MEK reagieren.

Da sowohl die chemische Umsetzung/Zerstörung als auch die einfache Rektifikation ausscheiden, wurde versucht, durch eine Rektifikation mit Hilfsstoffen (Extraktiv-Rektifikation) eine Trennung zwischen den Schlüsselkomponenten Diacetyl einerseits und MEK/EtOAc andererseits zu erreichen.

Entsprechende Versuche wurden mit dem bereits in der Literatur zur Trennung von EtOH und EtOAc vorgeschlagenen Ethylenglykol in einer Extraktivrektifikation gefahren.

Als Apparatur diente eine kleine, kontinuierlich beschickte Füllkörper-Kolonne.

Durchmesser: 15 mm

Höhe der Schüttung: 150 cm, unterteilt in 6 Schüsse

Füllkörper: Drahtwendeln (2-4 mm Durchmesser)

Bodenzahl: ca. 30 theoretische Böden, getestet mit EtOH/IPA

Zulaufmenge an FEED: 25 g/h (oberhalb des 2. Schusses)

Zulauf an Extraktivstoff: (oberhalb des 5. Schusses)

Gewichtsverhältnis Extraktivstoff (Zusatz) : Feed 8:1

Destillatmenge: 14 g/h ( ca. 56 Gew% des FEEDs)[*]

Rücklaufverhältnis

(am-Kopf, ohne Berücksichtigung des Zusatzes) ≈ 1:3

[*] entspricht mengenmäßig dem Gehalt an EtOAc + MEK

Als Ergebnis wurde erhalten:

| Stoff | Konzentrationen Gew% | |
|---|---|---|
| | im Feed | im Destillat |
| EtOH | 19.5 | 1 { 2 %}*) |
| Diac | 0.23 | – – |
| MEK | 28.8 | 30 {30 %} |
| EtOAc | 31.1 | 69 {95 %} |
| Etoxy | 2.3 | – – |
| Toluol | 1.2 | – – |
| H2O | 13.3 | 0.1 |
| | Sumpftemperatur 135°C | |

*) Die geschweifte Klammer gibt an, wieviel Gew.-% der im Zulauf enthaltenen Komponenten im Destillat erschienen sind.

Die Versuche mit Ethylenglykol zeigen zunächst das erwartete Ergebnis, daß sich nämlich EtOAc als Kopfprodukt abtrennen läßt und MEK vornehmlich in den Sumpf der Kolonne wandert. Das Diacetyl verbleibt bei dem MEK und Alkohol. Es ergaben sich jedoch hohe Bilanzverluste für MEK (ca. 30 % der eingesetzten Menge), was auf die Reaktion des Ethylenglykols mit MEK zum entsprechenden Ketal zurückzuführen ist. Deshalb entfällt beispielsweise die Verwendung von Ethylenglykol als Hilfsstoff für die Extraktivdestillation.

Anmerkung:

Dieses Beispiel für die Extraktivrektifikation beweist, daß die Anwesenheit des Diacetyls und seine Abtrennung für das beschriebene Stoffgemisch weitaus höhere Anforderungen stellt als die bekannte Trennung von MEK, EtOAc und Alkohole, für die Extraktivrektifikationen mit Glykolen vorgeschlagen werden.

8

| V26 | Gew-% | g/h | Gew-% | g/h | Gew% des Zulaufs[1] |
|---|---|---|---|---|---|
|  | Organ.Phase | | wäss. Phase | | |
| Destillat | | 313.2 | | 20.1 | 9.67 |
| EtOH | 0.0 | 0.0 | 0.0 | 0.0 | 0.0 |
| IPA | 0.03 | 0.09 | 0.02 | 0.004 | 0.006 |
| Diac | 0.01 | 0.04 | 0.0 | 0.0 | 2.4 |
| MEK | 43.48 | 136.18 | 9.79 | 1.96 | 91.3 |
| EtOAc | 49.11 | 153.81 | 4.61 | 0.92 | 98.25 |
| Etoxy | 0.01 | 10.04 | 0.0 | 0.0 | 0.003 |
| Tol | 2.30 | 7.2 | 0.0 | 0.0 | 87.9 |
| H2O | 5.05 | 15.82 | 85.59 | 17.7 | 1.10 |

Rücklauf: 2666 g/h
Rücklaufverhältnis: 8:1

72.5°C

Extraktivstoff 6:1
2944 g/h Wasser

90 Glockenböden

≈ 50–60 theoret. Trennstufen

Durchmesser = 40 mm

100°C

| V26 | Gew-% | g/h |
|---|---|---|
| Feed=Zulauf | | 502.81 |
| EtOH | 21.53 | 108.3 |
| IPA | 2.95 | 14.8 |
| Diac | 0.31 | 1.6 |
| MEK | 30.09 | 151.3 |
| EtOAc | 31.32 | 157.5 |
| Etoxy | 2.69 | 13.5 |
| Tol | 1.63 | 8.2 |
| H2O | 9.48 | 47.7 |

Betriebszeit: 41 Stunden im stationären Betriebszustand

Analysen von Destillat und Sumpf sind Mittelwerte aus 14 entnommenen Proben.

tief gelb; Diacetylanreicherung! Abziehen des Seitenstromes leicht möglich

| V26 | Gew-% | g/h | Gew% des Zulaufs[1] |
|---|---|---|---|
| Sumpfabzug | | 3093.7 | 89.8 |
| EtOH | 3.64 | 112.61 | 104.0 |
| IPA | 0.50 | 15.5 | 104.3 |
| Diac | 0.05 | 1.48 | 94.9 |
| MEK | 0.26 | 8.04 | 5.3 |
| EtOAc | 0.0 | 0.0 | 0.0 |
| Etoxy | 0.43 | 13.3 | 98.3 |
| Tol | 0.0 | 0.0 | 0.0 |
| H2O | 95.12 | 2942.6 | 98.4 |

[1] Bezogen auf Wasserzulauf + Feed ( = 3446.7 g/h)

Schaubild 1: Kolonne K1: Extraktivrektifikation mit Wasser zur Abtrennung des Diacetyls von MEK/EtOAc

3) Verfahrensstufe Extraktivrektifikation (K1)

Es zeigte sich, daß überraschenderweise das Diacetyl quantitativ von MEK und EtOAc durch eine Extraktivrektifikation mit Wasser abgetrennt wurde, wenn während der Destillation kontinuierlich eine ausreichende Wassermenge zusätzlich auf den Kopf der Kolonne gefahren wurde.

In einem mehrtägigen kontinuierlichen Dauerversuch wurden die im obigen Schaubild 1 dargestellten Ergebnisse erhalten.

Kurz oberhalb des Sumpfes war die Lösung auf den Böden durch die Anreicherung von Diacetyl tiefgelb gefärbt, so daß sich ein Seitenstromabzug des angereicherten Diacetyls anbietet.

4) Verfahrensstufe Wasserrückgewinnung (K2)

Aus dem Sumpfablauf der Extraktivrektifikation werden die organischen Lösungsmittel über Kopf abgetrieben, wie es der Kolonne K(2) im Fließschema Abb. 1 oder 2 entspricht; im Sumpf verbleibt nur Wasser.

Die Bedingungen und Ergebnisse des 2. Dauerversuches zeigt das folgende Schaubild 2.

| V28 | Gew-% | g/h | Gew% des Zulaufs |
|---|---|---|---|
| Destillat | | 42.1 | 8.3 |
| | | | |
| EtOH | 40.05 | 16.17 | 97.1 |
| IPA | 5.89 | 2.48 | 98.4 |
| Diac | 0.45 | 0.19 | 90.5 |
| MEK | 3.14 | 1.32 | 108.2 |
| EtOAc | 0.22 | 0.o9 | -.- |
| Etoxy | 5.68 | 2.39 | 112.2 |
| Tol | 0.01 | 0.01 | -.- |
| H2O | 44.55 | 18.75 | 3.9 |

Rücklauf: 337 g/h
Rücklaufverhältnis: 8:1

91 ± 1°C

| V28 | Gew-% | g/h |
|---|---|---|
| Feed=Zulauf | | 506.5 |
| | | |
| EtOH | 3.43 | 17.37 |
| IPA | 0.50 | 2.52 |
| Diac | 0.04 | 0.21 |
| MEK | 0.24 | 1.22 |
| EtOAc | <0.01 | 0.04 |
| Etoxy | 0.42 | 2.13 |
| Tol | 0.0 | 0.0 |
| H2O | 95.36 | 482.98 |

90 Glockenböden

≈ 50–60
theoret.
Trennstufen

Durchmesser
= 40 mm

Betriebszeit: 7 Stunden im stationären
Betriebszustand

Analysen von Destillat und Sumpf sind
Mittelwerte aus 3 entnommenen
Proben (Stundenmittel aus den
letzten drei Stunden)

≈ 99°C

| V28 | Gew-% | g/h | Gew% des Zulaufs |
|---|---|---|---|
| Sumpfabzug | | 462.2 | 91.3 |
| | | | |
| EtOH | <0.01 | 0.01 | 0.06 |
| IPA | 0.0 | 0.0 | 0.0 |
| Diac | <0.01 | 0.01 | 4.76 |
| MEK | 0.0 | 0.0 | 0.0 |
| EtOAc | 0.0 | 0.0 | 0.0 |
| Etoxy | <0.01 | 0.01 | 0.47 |
| Tol | 0.0 | 0.0 | 0.0 |
| H2O | 99.95 | 462.2 | 95.25 |

Schaubild 2: Kolonne K2: Regeneration des Kreislaufwassers
durch Abdestillieren der organischen Bestandteile

### 5) Verfahrensstufe Diacetyl-Zerstörung

Das Destillat S(11), bestehend aus den organischen Komponenten EtOH/IPA/Etoxy/Diacetyl und Wasser der Wasserrückgewinnung wird chemisch behandelt, um das Diacetyl in eine nichtflüchtige Substanz zu überführen.

Für die chemische Umsetzung des Diacetyls kommen in Frage:

1. Hydroxylamin
2. Natriumhydrogensulfit $NaHSO_3$
3. NaOH
4. Natriumcarbonat $Na_2CO_3$
5. Natriumbicarbonat $NaHCO_3$
6. $Ca(OH)_2/CaCO_3$

#### a) Hydroxylamin.

Die Umsetzung der diacetylhaltigen Lösung S(11) lieferte eine farblose Lösung. Die dabei eingetretene Bildung von Dimethylglyoxim wurde mit Nickel als rote Fällung nachgewiesen.

Diese Reinigungsoperation kommt nur in Frage, wenn keine anderen Ketone (MEK) mehr vorhanden sind.

#### b) Natriumhydrogensulfit.

Die diacetylhaltige Lösung (Kopfprodukt von Kolonne K(2) aus V28 (siehe Schaubild 2)) setzt sich zwar mit Natriumhydrogensulfit in der Kälte um; bei der anschließenden Rektifikation tritt allerdings wieder Rückspaltung mit $SO_2$-Geruch auf. Die Rückspaltung konnte durch Abdestillieren im Vakuum vermieden werden.

#### c) NaOH.

Die Zugabe von Natronlauge führt bereits bei Zimmertemperatur zur starken Schwarzfärbung der Lösung, aus der sich nach kurzzeitiger Erwärmung alle Wertprodukte (EtOH, IPA und Etoxy) quantitativ abdestillieren ließen. Weder im Destillat, noch im Sumpf ließ sich nach der Destillation Diacetyl nachweisen.

In Gegenwart von EtOAc führte die gleiche Maßnahme zur quantitativen Umsetzung des Alkalis infolge Verseifung des Esters unter Bildung von EtOH und Natriumacetat, ohne daß der Diacetylgehalt wesentlich abnahm.

#### d) Natriumcarbonat.

Die Reaktion verläuft bei Raumtemperatur langsamer als mit NaOH. Erst beim Erwärmen färbte sich die Lösung durch Umsetzung des Diacetyls schwarz; im Destillat war stets noch Diacetyl nachzuweisen.

In Gegenwart von EtOAc führt auch diese Reaktion zur teilweisen Verseifung des Esters unter Bildung von EtOH und Natriumacetat.

#### e) Natriumhydrogencarbonat.

Die Reaktion verläuft bei Raumtemperatur noch langsamer als mit Natriumcarbonat. Die Lösung verfärbte sich wiederum beim Sieden schwarz als Zeichen der eingetretenen Umsetzung; im Destillat war Diacetyl stets jedoch noch eindeutig nachzuweisen.

EtOAc wurde nicht in nennenswertem Maße verseift.

#### f) $Ca(OH)_2/CaCO_3$.

Es trat auch hier eine schwarze Verfärbung beim Abdestillieren auf, aber auch vorhandenes EtOAc ist quantitativ gespalten worden. Im Sumpf kam es gegen Ende der Destillation zu starkem Schäumen.

Zusammenfassend läßt sich feststellen:

Diacetyl wird am besten durch Kochen mit NaOH quantitativ zersetzt. Die Reaktion kann allerdings weder in der Rohlösung noch in der Kolonne K(1) während der Extraktivrektifikation ausgeführt werden, da es dort zur bevorzugten Umsetzung der Natronlauge mit EtOAc kommt.

Führt man die Umsetzung erst während der Aufarbeitung des Stromes S(11) aus, dann erhält man sogar die in diesem Strom enthaltenen Alkohole und das Etoxy völlig frei von Diacetyl als recyclingfähiges Material, wie es das folgende Beispiel zeigt:

Diacetyl-Versuch V35; diskontinuierliche Destillation

| Komponen. | [g] Einsatz | [g] Sumpf | [g] Destillat |
|---|---|---|---|
| EtOH | 16.8 | 0.005 | 17.8 |
| IPA | 2.8 | 0 | 2.9 |
| Diacetyl | 0.17 | 0.0 | 0.0 |
| MEK | 1.62 | 0.001 | 1.66 |
| Etoxy | 2.7 | 0.06 | 2.5 |
| $H_2O$ | Rest | Rest | Rest |

## 6) Verfahrensstufe Alkoholkonzentrierung

Für die Absolutierung von diacetylfreiem EtOH/IPA durch azeotrope oder extraktive Entwässerung sind gängige Routineverfahren bekannt. Sie sind nicht Gegenstand der Erfindung.

## 7) Verfahrensstufe Extraktion mit Extraktrückführung (Trennung von MEK/EtOAc durch Gegenstromextraktion mit Wasser)

Das Destillat S(5) aus der Extraktivrektifikation in Kolonne K(1) wird in einer Gegenstromextraktion (mit Extraktrücklauf) mit Wasser als Lösungsmittel in ein wassergesättigtes Raffinat (EtOAc/Toluol) und in einen wassergesättigten Extrakt (MEK:EtOAc = 80:20) zerlegt.

Der in der Extraktivrektifikation mit Wasser in der ersten Kolonne (K1) abgezogene und kondensierte Seitenstrom S(5) enthielt

| | Gew-% | g/h | Gew-% | g/h | Gew% des Zulaufs |
|---|---|---|---|---|---|
| | Organ.Phase | | wäss. Phase | | |
| Destillat | | 313.2 | | 20.1 | 9.67 |
| EtOH | 0.0 | 0.0 | 0.0 | 0.0 | 0.0 |
| IPA | 0.03 | 0.09 | 0.02 | <0.01 | 0.006 |
| Diac | 0.01 | 0.04 | 0.0 | 0.0 | 2.4 |
| MEK | 43.48 | 136.18 | 9.79 | 1.96 | 91.3 |
| EtOAc | 49.11 | 153.81 | 4.61 | 0.92 | 98.25 |
| Etoxy | 0.01 | 10.04 | 0.0 | 0.0 | 0.003 |
| Tol | 2.30 | 7.2 | 0.0 | 0.0 | 87.9 |
| $H_2O$ | 5.05 | 15.82 | 85.59 | 17.7 | 1.10 |
| Extraktionszulauf (Destillat der Kolonne K1) | | | | | |

Während die wäßrige Phase des Kondensats S(5) als Rücklauf in die Kolonne zurückgeführt wurde, ist das MEK, das EtOAc und Toluol in der organischen Phase weiter aufgetrennt worden. Es wurde gefunden, daß sich dazu eine Gegenstromextraktion mit Wasser eignet, wenn man durch eine teilweise Rückführung von angereichertem Extrakt die Konzentrationsverhältnisse in der Extraktion so einstellt, daß der anfallende Extrakt die beiden Komponenten MEK und EtOAc im Verhältnis 80:20 enthält. Für dieses Gemisch bestehen viele Verwendungsmöglichkeiten. Als Raffinat konnte dann ein EtOAc mit ca. 1 % MEK erhalten werden, das den Anforderungen in zahlreichen Industriezweigen genügt.

Die Anwesenheit von geringen Toluol-Mengen (< 2 Gew.-% in der organischen Phase) hatte auf den Verlauf der Extraktion und die sich einstellenden Verteilungen keinen nennenswerten Einfluß. Bei der Extraktion mit Wasser verbleibt unter den genannten Bedingungen das Toluol, wie die ausführlichen Versuche der nachfolgend aufgeführten Tabelle zeigen, praktisch quantitativ in der Raffinatphase.

## Tabelle I: Verteilung von Toluol bei der Extraktion zwischen den beiden Extraktionsphasen

### Konzentrationen in Gew-%

| organische Phase | | | | | | wäßrige Phase | | | | | |
|---|---|---|---|---|---|---|---|---|---|---|---|
| Toluol | MEK | EtOAc | EtOH | IPA | Wasser | Toluol | MEK | EtOAc | EtOH | IPA | Wasser |
| 0.60 | 6.86 | 75.15 | 14.31 | - | 3.08 | - | 2.30 | 12.90 | 16.07 | - | 68.73 |
| 0.60 | 18.20 | 60.30 | 14.89 | - | 6.01 | - | 6.60 | 12.50 | 15.22 | - | 65.68 |
| 0.50 | 24.90 | 38.00 | 15.50 | - | 21.10 | - | 14.50 | 17.80 | 15.46 | - | 52.24 |
| 14.33 | 23.65 | 59.23 | 0.07 | 0.03 | 2.69 | - | 12.08 | 3.59 | 0.82 | 1.13 | 82.38 |
| 14.66 | 23.63 | 58.92 | 0.07 | 0.05 | 2.67 | - | 12.14 | 3.56 | 0.83 | 1.12 | 82.36 |
| 28.89 | 12.80 | 54.85 | 0.02 | 0.0005 | 2.44 | 0.04 | 2.55 | 4.05 | 0.07 | 0.01 | 93.28 |

Die Versuche ergaben weiterhin, daß keine nennenswerte Konzentration an EtOH im Extraktionszulauf vorhanden sein darf, da EtOH als Lösungsvermittler zwischen EtOAc, MEK und Wasser die Extraktion eines 20:80-Gemisches aus MEK/EtOAc verhindert. So erlaubt die Anwesenheit von 15 Gew.-% EtOH, daß die Extraktion bestenfalls ein Gemisch aus MEK und EtOAc im Verhältnis von 50:50 Gew.-% liefert.

FEED

WASSER

Extraktabzug

Trennung durch
Rektifikation
(Entwässerung)

RAFFINAT
(EtOAC/H2O)

MEK/EtOAc
(+H2O)

WASSER

(zur Extraktion, bzw.
Extraktivrektifikation)

**Schaubild 3:** Fließbild der extraktiven Trennung in
MEK/EtOAc und EtOAc

In einer 11-stufigen Mischer-Scheider-Batterie wurde die Extraktion im Gegenstrom mit Feedzuführung zum 3. Scheidetrichter ausgeführt (siehe Schaubild 3), wobei jeweils ein Scheidetrichter eine theoretische Trennstufe verwirklicht, da bei intensivem Schütteln ein Wirkungsgrad von 100 % erreicht wird.

Die Zulaufkonzentrationen waren entsprechend dem Destillat aus Kolonne K(1) = FEED und dem Destillat aus Kolonne K(3) = EXTRAKTRÜCKFÜHRUNG eingestellt worden.

Die Versuchsbedingungen und Ergebnisse dieser Extraktion, wie sie der nachstehenden Tabelle II zu entnehmen sind, beweisen, daß

1. ein wäßriger Extrakt erhalten wurde, der 4,2 g EtOAc auf 13,8 g MEK enthielt; entsprechend einem MEK mit ∿20 Gew.-% EtOAc;

2. Ein Raffinat erzeugt worden ist, das 0,8 g MEK auf 96 g EtOAc beinhaltet; entsprechend einem EtOAc mit <1 Gew.-% MEK.

15

## Tabelle II: Ergebnisse des Extraktionsversuches

|  | Gew% MEK | Gew% EtOAc | Gew% H2O | g/Zyklus |
|---|---|---|---|---|
| Feed | 46.2 | 48.5 | 5.3 | 50 |
| Lösungsmittel | -.- | -.- | 100.0 | 342 |
| Rückführung | 75.5 | 18.9 | 5.6 | 45.2 |

|  | Gew% MEK | Gew% EtOAc | Gew% H2O | g/Zyklus |
|---|---|---|---|---|
| Raffinat | 0.8 | 96.1 | 3.1 | 18.8 |
| Extrakt gem. | 13.8 | 4.2 | 82.0 | 417.3 |

8) Verfahrensstufe Lösungsmittelrückgewinnung (K3)

Aus dem ca. 80 % Wasser enthaltenden Extrakt S(6) liefert die Rektifikation K(3) als Destillat ein mit Wasser gesättigtes Gemisch, bestehend aus MEK und EtOAc im Verhältnis (80:20). Der Sumpf S(10) dieser Kolonne K(3) besteht nurmehr aus dem Extraktions-Lösungsmittel Wasser. Die in dieser Rektifikation erhaltenen Ergebnisse eines Beispiels sind im Schaubild 4 aufgeführt (Versuchsergebnis der Kolonne K(3)). Nach dieser Vorentwässerung hat in einer nächsten Stufe die restliche Trocknung des Mischlösungsmittels MEK/EtOAc zu erfolgen.

| V29 | Gew-% | g/h | Gew-% | g/h | Gew% des Zulaufs |
|---|---|---|---|---|---|
| | Organ.Phase | | wäss. Phase | | |
| Destillat | | 83.52 | | 31.62 | 22.65 |
| EtOH | <0.01 | <0.01 | <0.01 | <0.01 | 0.0 |
| IPA | <0.01 | <0.01 | <0.01 | <0.01 | 0.0 |
| Diac | -.- | -.- | -.- | -.- | -.- |
| MEK | 72.80 | 60.80 | 19.50 | 6.16 | 93.26 |
| EtOAc | 18.64 | 15.57 | 2.40 | 0.76 | 95.72 |
| Etoxy | -.- | -.- | -.- | -.- | -.- |
| Tol | -.- | -.- | -.- | -.- | -.- |
| H2O | 8.55 | 7.14 | 78.09 | 24.69 | 7.59 |

Rücklauf: 921 g/h ↓ ↑
Rücklaufverhältnis: 8:1

71.0°C

90 Glockenböden

≈ 50-60 theoret. Trennstufen

Durchmesser = 40 mm

103°C

| V29 | Gew-% | g/h |
|---|---|---|
| Feed-Zulauf | | 508.44 |
| EtOH | <0.01 | <0.01 |
| IPA | <0.01 | <0.01 |
| Diac | -.- | -.- |
| MEK | 14.12 | 71.81 |
| EtOAc | 3.35 | 17.05 |
| Etoxy | -.- | -.- |
| Tol | -.- | -.- |
| H2O | 82.52 | 419.57 |

Analysen von Destillat und Sumpf sind Mittelwerte aus 3 entnommenen Proben.

| V29 | Gew-% | g/h | Gew% des Zulaufs |
|---|---|---|---|
| Sumpfabzug | | 386.12 | 75.94 |
| EtOH | 0.0 | 0.0 | 0.0 |
| IPA | 0.0 | 0.0 | 0.0 |
| Diac | -.- | -.- | -.- |
| MEK | <0.01 | 0.01 | 0.02 |
| EtOAc | 0.0 | 0.0 | 0.0 |
| Etoxy | -.- | -.- | -.- |
| Tol | -.- | -.- | -.- |
| H2O | 99.99 | 386.11 | 92.03 |

Schaubild 4: Kolonne 3: Wasserrückgewinnung für die Extraktion

9) Beispiele für die Entwässerung des Mischlösungsmittels

a) Selbstentwässerung durch das ternäre Azeotrop MEK/EtOAc/$H_2O$

Verschiedene Zusammensetzungen der drei Stoffe MEK, EtOAc und Wasser wurden in einer Kolonne jeweils 6 Stunden unter totalem Rücklauf destilliert. Dann erfolgte bei einem Rücklaufverhältnis von 30:1 die Entnahme einer Destillatprobe.

| Kolonnendaten: | |
|---|---|
| Schüttung | 2-4 mm Drahtwendeln |
| Schütthöhe | 1.5 m (in 6 Abschnitte unterteilt) |
| Trennleistung | ca. 30 theoret. Böden, gemessen mit ETOH/IPA |

Das Destillat war zweiphasig mit folgenden Zusammensetzungen:

| Komponente | Gew% | |
|---|---|---|
| | organische Phase | wäßrige Phase |
| MEK | 3.4 | 1.0 |
| EtOAc | 92.9 | 7.0 |
| H2O | 3.7 | 92.0 |

Der Anteil der wäßrigen Phase beträgt nur ca. 0,5 Gew.-% des Gesamtgemisches, so daß das Netto-Wasser-Schleppvermögen für die im US-Patent 2 649 407 vor geschlagene azeotrope Selbstentwässerung als unzureichend zu bewerten ist.

b) Heteroazeotrop-Rektifikation mit Hilfsstoff

Es wurde gefunden, daß tiefsiedende Äther, genauso wie das in DE-3 606 121 erwähnte Cyclohexan mit Wasser Azeotrope bilden, jedoch im Gegensatz zu Cyclohexan keine Azeotrope mit MEK und EtOAc auftreten. Die entsprechenden azeotropen Zusammensetzungen sind in der zuvor beschriebenen Apparatur und Weise wie folgt ermittelt worden:

| Komponente | °C | Organische Destillatphase | | | | |
|---|---|---|---|---|---|---|
| | | Phasenanteil | Gew% EtOAc | Gew% MEK | Gew% H2O | Gew% Lsgm. |
| Methyl-tert-butyläther | 52.0 | 97.7 | 0.0 | 0.0 | 1.45 | 98.46 |
| Diisopropyläther | 61.5 | 98.4 | 1.1 | 8.1 | 0.7 | 90.1 |
| Cyclohexan | 63.5 | 97.4 | 12.0 | 8.0 | 0.7 | 79.0 |

| Komponente | °C | wässerige Destillatphase | | | | | Netto-Wassertransport in % |
|---|---|---|---|---|---|---|---|
| | | Phasenanteil | Gew% EtOAc | Gew% MEK | Gew% H2O | Gew% Lsgm. | |
| Methyl-tert-butyläther | 52.0 | 2.3 | 0.0 | 0.0 | 94.5 | 5.5 | 2.3 |
| Diisopropyläther | 61.5 | 1.6 | 0. | 0. | 90 | <1 | 1.5 |
| Cyclohexan | 63.5 | 2.6 | 3.0 | 6.0 | 90 | - | 2.3 |

Der erstmals für diese Zwecke eingesetzte MTBE erweist sich als am günstigsten, da sein Azeotrop bei gleichem Nettowassertransport wie Cyclohexan um 11°C niedriger siedet.

EP 0 384 458 B1

Beispiel für die azeotrope Entwässerung mit MTBE Extraktentwässerung (≡ Kolonne 4)

Die Entwässerungsversuche wurden diskontinuierlich in einer Füllkörperkolonne folgender Abmessungen durchgeführt.

| Kolonnendaten: | Durchmesser | 15 mm |
| | Schüttung | 2 - 4 mm Drahtwendeln |
| | Schütthöhe | 1.25 m (unterteilt in 5 Schüsse) |
| | Trennleistung | ca. 25 theor. Böden (ermittelt bei EtOH/IPA) |

Entwässert wurden 1 l eines Gemisches, das mit Ausnahme des Wassergehaltes dem aufkonzentrierten und vorentwässerten Extrakt S(9) entsprach.
Zusammensetzung:
78.2 Gew-% MEK
20.2 Gew-% EtOAc
0.05 Gew-% Toluol
1.5 Gew-% Wasser
Die Wasserkonzentration war geringer gewählt worden, um den Entwässerungsvorgang in der kleinen Kolonne abzukürzen. Außerdem wurde Toluol in geringer Menge zugegeben, um zu überprüfen, ob es, unvorhergesehener Weise in den Extrakt gelangt,einen nachteiligen Einfluß auf die Entwässerung hat.
Am Kopf der Kolonne wurde ein Abscheider montiert, der vor Versuchsbeginn mit MTBE und Wasser gefüllt wurde. Zusätzlich wurden ca. 120 ml des Äthers in die Kolonne gegeben. Der Überlauf am Abscheider ließ einen Rückfluß der organischen Phase auf den Kopf der Kolonne zu.
Die Kolonne ist solange betrieben worden, bis die Wasserkonzentration im Sumpf unter 500 ppm lag. Dann wurde der Abscheider demontiert und ca. die Hälfte des Sumpfinhaltes in Fraktionen über Kopf abgezogen.
Die Zusammensetzung der entnommenen Destillatproben (in Gew.-%) war:

| Probe | MTBE | MEK | EtOAc | Toluol | Wasser |
|-------|--------|--------|--------|--------|--------|
| D 1 | 84.489 | 6.644 | 6.467 | - | 2.40 |
| D 2 | 23.245 | 49.621 | 26.471 | - | 0.663 |
| D 3 | 9.449 | 64.955 | 25.052 | - | 0.544 |
| D 4 | 3.667 | 70.834 | 25.182 | - | 0.317 |
| D 5 | 1.120 | 74.689 | 24.030 | - | 0.161 |
| Sumpf | 0.026 | 85.591 | 14.259 | 0.11 | 0.014 |

Die Destillationsergebnisse lassen erkennen, daß eine Abtrennung des Wassers vom Mischlösungsmittel mit MTBE möglich ist und auch die Abtrennung des MTBE selbst aus dem entwässerten Extrakt keine Schwierigkeiten bereitet.

10) Entwässerung des Raffinates in Kolonne K(5)

Die Entwässerungs-Versuche wurden in einer Füllkörperkolonne

| Durchmesser | 15 mm |
| Schüttung | 2 - 4 mm Drahtwendeln |
| Schütthöhe | 1.25 m (unterteilt in 5 Schüsse) |
| Trennleistung | ca. 25 theor. Böden (ermittelt bei EtOH/IPA) |

ausgeführt, deren Sumpf mit ca. 1 l eines Gemisches aus:
1 Gew-% MEK
94 Gew-% EtOAc
2 Gew-% Toluol
3 Gew-% Wasser
gefüllt war. Diese Zusammensetzung entspricht dem Raffinat, das die Extraktion verläßt. Zweck dieses Versuches war es zu zeigen, daß sich EtOAc mit MTBE entwässern läßt und daß sich MTBE von EtOAc auch dann noch im Sumpf abtrennen läßt, wenn kein Wasser mehr vorhanden ist (keine Bildung von Azeotropen).

An die Kolonne wurde ein Abscheider montiert, der vor Versuchsbeginn mit MTBE und Wasser gefüllt wurde. Zusätzlich wurden ca. 120 ml des Äthers in die Kolonne gegeben. Der Überlauf am Abscheider ließ einen Rückfluß der organischen Phase auf den Kopf der Kolonne zu.

Die Kolonne ist solange betrieben worden, bis die Wasserkonzentration im Sumpf unter 500 ppm lag. Dann wurde der Abscheider demontiert und ca. die Hälfte des Sumpfinhaltes in Fraktionen über Kopf abgezogen.

Die Konzentrationen der entnommenen Proben in Gew.-% enthält die folgende Tabelle.

| Probe | MTBE | MEK | EtOAc | Toluol | Wasser |
|---|---|---|---|---|---|
| D 1 | 81.207 | 0.161 | 17.022 | - | 1.61 |
| D 2 | 5.222 | 0.928 | 90.770 | - | 3.08 |
| D 3 | 0.839 | 1.179 | 94.803 | - | 3.18 |
| D 4 | 0.145 | 1.026 | 97.459 | - | 1.37 |
| Sumpf | - | 0.879 | 95.078 | 4.036 | 0.007 |

Die Destillate D1 bis D3 waren zweiphasig, wobei die Tabelle nur die Analyse der organischen Phase enthält, da der Anteil wäßriger Phase verhältnismäßig gering war.

Aufgrund der Analyse des Sumpfinhaltes nach Beendigung des Versuches läßt sich feststellen, daß das Sumpfprodukt der Raffinatentwässerung bei entsprechender Gestaltung der Rektifikationskolonne ätherfrei erhalten werden kann.

## 11) Verfahrensstufe Toluolabtrennung (K6)

Das Sumpfprodukt der Raffinatentwässerung aus Kolonne K(5) läßt sich durch eine einfache Rektifikation in wasserfreies EtOAc und Toluol trennen. Geringe Mengen MEK stören diese Destillation nicht.

Beispiel:

In einer Füllkörperkolonne

| | |
|---|---|
| Durchmesser | 15 mm |
| Schüttung | 2 - 4 mm Drahtwendeln |
| Schütthöhe | 1.25 m (unterteilt in 5 Schüsse) |
| Trennleistung | ca. 25 theor. Böden (ermittelt bei EtOH/IPA) |

wurde ein Gemisch aus EtOAc, Toluol und MEK, wie es dem Sumpfprodukt der Raffinatentwässerung entspricht, in einer diskontinuierlichen Rektifikation in Fraktionen zerlegt.

Zusammensetzung des eingesetzten Anfangsgemisches:

1 Gew-% MEK

97 Gew-% EtOAc

2 Gew-% Toluol

0.03 Gew-% Wasser

Bei einem Rücklaufverhältnis von 2:1 wurden 4 Destillatproben entnommen. Die Zusammensetzung dieser Fraktionen und die des Sumpfinhaltes nach Versuchsende enthält die Tabelle.

| Probe | MEK | EtOAc | Toluol | Wasser |
|-------|------|--------|--------|--------|
| D 1 | 1.630 | 98.173 | - | 0.18 |
| D 2 | 1.367 | 98.584 | - | 0.04 |
| D 3 | 1.284 | 98.686 | - | 0.03 |
| D 4 | 1.248 | 98.732 | - | 0.02 |
| Sumpf | 0.909 | 96.609 | 2.48 | 0.003 |

Alle Destillate waren toluolfrei. Dieser Versuch zeigt deutlich, daß sich Toluol und EtOAc in einer einfachen Rektifikation gut trennen lassen.

## Patentansprüche

**1.** Verfahren zur Trennung von diacetylhaltigen Gemischen aus Methylethylketon, Ethylacetat, Ethanol und Wasser, die gegebenenfalls Toluol, Isopropanol, Essigsäure und Kohlenwasserstoffe, insbesondere n-Hexan, enthalten, unter Bildung von Methylethylketon (mit etwa 20 Gew.-% Ethylacetat, höchstens 0,5 Gew.-% Ethanol und höchstens 0,1 Gew.-% Wasser), Ethylacetat (mit höchstens 1 Gew.-% Methylethylketon, höchstens 0,5 Gew.-% Ethanol und höchstens 0,05 Gew.-% Wasser) und Ethanol (mit höchstens 0,1 Gew.-% Methylethylketon, höchstens 1 Gew.-% Wasser und höchstens 1 Gew.-% Ethylacetat) von Lösungsmittelqualität zur Wiederverwendung,
**gekennzeichnet** durch die folgende Kombination von Verfahrensmaßnahmen:
a) die in dem Ausgangsgemisch gegebenenfalls enthaltene Essigsäure wird durch Neutralisation mit Natriumcarbonat eliminiert;
b) das durch Farbe und Geruch störende, in dem neutralisierten Ausgangsgemisch enthaltene Diacetyl wird durch Extraktiv-Rektifikation mit Wasser und nachfolgende Umsetzung mit Alkali- oder Erdalkalihydroxiden, Alkali- oder Erdalkalicarbonaten oder Oximen entfernt oder als unterer Seitenstrom in aufkonzentrierter Form kurz oberhalb des bei der Extraktiv-Rektifikation entstehenden Sumpfes abgezogen;
c) das bei der Extraktiv-Rektifikation in der Stufe (b) entstehende Destillat, das frei von Alkoholen sein soll, wird als oberer Seitenstrom abgezogen und durch Gegenstrom-Extraktion mit Extraktrückführung in ein Mischlösungsmittel aus etwa 80 Gew.-% Methylethylketon und etwa 20 Gew.-% Ethylacetat (wasserfrei gerechnet) und in nahezu wasserfreies Ethylacetat zerlegt;
d) die Entfernung des restlichen Wassers aus dem bei der Gegenstrom-Extraktion in der Stufe (c) entstehenden Raffinat erfolgt durch azeotrope Entwässerung mittels Äthern oder Kohlenwasserstoffen, deren azeotrope Siedepunkte mit Wasser unterhalb 70°C liegen;
e) Auftrennung des in der Stufe (d) erhaltenen Sumpfprodukts durch Rektifikation in praktisch reines Ethylacetat und praktisch reines Toluol;
f) Rückgewinnung und Rückführung der Hauptmenge des als Lösungsmittel für die Gegenstrom-Extraktion dienenden Wassers aus dem Extrakt der Gegenstrom-Extraktion durch Abdestillieren und Rückführung eines Teils der organischen Phase des dabei entstehenden kondensierten ternären Methylethylketon-Ethylacetat-Wasser-Gemisches in die Gegenstrom-Extraktion; und
g) Entfernung des restlichen Wassers aus dem ternären Methylethylketon-Ethylacetat-Wasser-Gemisch durch azeotrope Entwässerung mittels Äthern oder Kohlenwasserstoffen, deren azeotrope Siedepunkte mit Wasser unterhalb 70°C liegen, unter Bildung eines Sumpfprodukts aus Methylethylketon und Ethylacetat im Gewichtsverhältnis 80:20.

**2.** Verfahren nach Anspruch 1, dadurch gekennzeichnet, daß
a) die Entfernung des restlichen Wassers aus dem Raffinat der Gegenstrom-Extraktion in der Stufe (d) des Anspruches 1 durch azeotrope Entwässerung mittels Äthern oder Kohlenwasserstoffen (vorzugsweise unter Verwendung von Cyclohexan, n-Hexan und insbesondere Methyl-tert-butyläther) durchgeführt wird, wobei deren azeotrope Siedepunkte mit Wasser unterhalb 70°C liegen sollen;
b) die Rückführung des ternären Gemisches Methylethylketon-Ethylacetat-Wasser gemäß Stufe (f) des Anspruches 1 in eine Extraktionsstufe am Extraktionsende erfolgt;
c) der Zulauf (Feed) innerhalb der Extraktion (beispielsweise in die dritte Stufe von insgesamt 11 Stufen) zwischen dem Extraktionsende und dem Extraktionsanfang erfolgt;
d) die Entfernung des restlichen Wassers aus dem ternären Gemisch Methylethylketon-Ethylacetat-Wasser gemäß Stufe (g) des Anspruches 1 durch azeotrope Entwässerung mittels Äthern oder Kohlenwasserstoffen (vorzugsweise unter Verwendung von Cyclohexan, n-Hexan und insbesondere Methyl-tert-butyl-äther) durchgeführt wird, deren azeotrope Siedepunkte mit Wasser unterhalb 70 °C liegen sollen, wobei für die Entfernung des restlichen Wassers aus dem Raffinat [Ethylacetat/-(Toluol)/Wasser] und aus dem roh entwässerten Extrakt [ternäres Gemisch Methylethylketon-Ethylacetat-Wasser] vorzugsweise das gleiche Schleppmittel [Äther,Kohlenwasserstoffe] eingesetzt wird;

e) die wässerige Phase der in der obigen Stufe (d) beschriebenen Entwässerung die Wasserrückgewinnung (Kolonne 3) eingespeist wird und von dort mit dem abdestillierten Destillat (S7) [ternäres Gemisch Methylethylketon-Ethylacetat-Wasser] wieder in die in der obigen Stufe (d) beschriebene Restentwässerung ohne Schleppmittelverlust gelangt; und

f) nach der Umsetzung des Diacetyls in der verbleibenden wäßrigen Lösung der Alkohole, vorzugsweise Ethanol und Isopropanol, diese Alkohole durch Destillation zurückgewonnen werden.

3. Verfahren anch Anspruch 1 oder 2, dadurch gekennzeichnet, daß zur Trennung eines Ausgangsgemisches (Feed), bestehend aus

etwa 25 bis 30 Gew.-% Methylethylketon (MEK)

etwa 25 bis 35 Gew.-% Ethylacetat (EtOAc)

etwa 20 bis 25 Gew.-% Ethanol (EtOH)

etwa 10 bis 15 Gew.-% Wasser ($H_2O$)

etwa 1 Gew.-% Isopropanol (IPA)

etwa 0,5 Gew.-% Toluol (Tol)

etwa 0,2 Gew.-% Kohlenwasserstoffen, vorzugsweise n-Hexan (Hex)

etwa 2,5 Gew.-% Ethoxypropanol und Propylenglycolmethyläther (Etoxy)

etwa 0,2 Gew.-% Essigsäure (AcOH) und

etwa 0,2 Gew.-% Diacetyl (Diac)

in wiederverwendbares Methylethylketon (mit etwa 20 Gew.-% EtOAc, höchstens 0,5 Gew.-% EtOH und höchstens 0,1 Gew.-% $H_2O$), Ethylacetat (mit höchstens 1 Gew.-% MEK, höchstens 0,5 Gew.-% EtOH und höchstens 0,05 Gew.-% $H_2O$) und Ethanol (mit höchstens 1 Gew.-% MEK, höchstens 1 Gew.-% $H_2O$ und höchstens 1 Gew.-% EtOAc) von Lösungsmittelqualität

a) das Ausgangsgemisch (Feed) durch Zugabe von Natriumcarbonat neutralisiert wird zur Eliminierung der darin enthaltenen Essigsäure,

b) der neutralisierte Zulauf Feed S (1) durch Extraktiv-Rektifikation in einer Rektifikationskolonne K (1) mit einem zurückgeführten Wasserstrom S (4) in die drei Ströme S (2), S (3) und S (5) zerlegt wird,

c) am Kopf der Kolonne K (1) ein Gemisch S (3) aus etwa 1/4 MEK und etwa 3/4 n-Hexan (mit etwa 1 Gew.-% Wasser) als obere Phase in einem Phasenabscheider A (1) abgezogen wird, wobei die untere wäßrige Phase in die Kolonne zurückfließt, und das anschließend getrocknete Lösungsmittelgemisch aus n-Hexan und MEK der weiteren Verwendung zugeführt wird;

d) mit dem Sumpf S (2) der Kolonne K (1) das gesamte Diacetyl zusammen mit EtOH, IPA, Etoxy (sowie eventuell vorhandenen geringen Mengen Natriumacetat aus der Essigsäureneutralisationsstufe (a)) in wäßriger Lösung abgezogen wird unter weitgehender Entfernung von MEK und EtOAc aus dem Sumpfablauf oder

e) das Diacetyl, das sich kurz oberhalb des Sumpfes von K (1) angereichert hat, als unterer Seitenstrom in aufkonzentrierter Form abgezogen wird;

f) als oberer Seitenstrom aus der Kolonne K (1) ein Destillat S (5) abgezogen wird, das - frei von Diacetyl und auch frei von Alkoholen - etwa je zur Hälfte aus MEK und EtOAc neben 5 Gew.-% Wasser und etwa 1 Gew.-% Toluol besteht,

g) als Rektifikations-Hilfsmittel Wasser S (4) verwendet wird, das ein Regenerat (Teil des Sumpfabzugs) aus weiter unten beschriebenen Entwässerungskolonnen K (2) und K (3) ist, wobei geringe Restgehalte an MEK und EtOAc nicht stören;

h) in einer Kolonne K (2) aus dem Sumpfprodukt S (2) der Kolonne K (1) der Hilfsstoff Wasser für die Kreisläufe zurückgewonnen wird, indem ein Gemisch aus EtOH/IPA/Etoxy/Diacetyl/Wasser als Destillat S (11) abgezogen wird, wobei der Sumpfstrom S (12) zur Extraktiv-Rektifikation in der Kolonne K (1) und als Lösungsmittel S (27) bzw. S (14) der weiter unten beschriebenen Gegenstrom-Extraktion dient und ein dem Wassergehalt des Feed und der Frischwasserzufuhr zum Lösungsmittel S (14) entsprechende Menge S (26) des Stromes S (27) ausgeschleust wird, wodurch auch das in geringen Mengen bei der Neutralisation gebildete Natriumacetat, falls es nicht schon zuvor aus dem Zulauf der Kolonne K (1) entfernt worden war, abgeführt wird;

i) das Destillat S (11) auf an sich bekannte Weise durch Alkoholabsolutierung vom Wasser befreit wird, wobei gleichzeitig das Diacetyl durch Zugabe kleiner Mengen Alkalihydroxid oder Alkalicarbonat durch Kochen zerstört oder durch Reagentien wie Hydroxylamin, Alkalisulfit und dgl. chemisch gebunden wird, wodurch Diacetyl ohne Verluste durch Verseifung von EtOAc oder durch Umsetzung von MEK, die beide bereits aus dem Ausgangsgemisch entfernt worden sind, dem Gemisch entzogen wird durch Ausschleusen des mit tiefschwarzen Zersetzungsprodukten des Diacetyls in sehr geringerMenge als Sumpfprodukt anfallenden Abwassers;

j) das Destillat S (5) zu der mit Wasser S (14) als Lösungsmittel ausgeführten Gegenstromextraktion mit Extraktrücklauf S (8) geführt wird;

k) in der Gegenstrom-Extraktion mit Extraktrücklauf als Extrakt eine wässerige Lösung S (6) erhalten wird, die etwa 83 Gew.-% Wasser enthält und dieMEK und EtOAc im Verhältnis von mindestens 4:1 enthält, während als Raffinat S (15) ein Gemisch aus etwa 93 Gew.-% EtOAc und etwa 3 Gew.-% Wasser sowie praktisch dem gesamten Toluol (etwa 2 Gew.-%) erhalten wird,

l) die Menge des Extraktionsmittels Wasser und der Extraktrücklauf so aufeinander abgestimmt werden, daß der MEK-Gehalt des Raffinats 1 Gew.-% nicht übersteigt, um die Reinheitsanforderungen des EtOAc zu erfüllen;

m) der Extraktstrom S (6) und die wässerige Phase S (21) aus dem Abscheider A(2) in der Vorentwässerungskolonne K (3) aufgearbeitet werden unter Bildung eines Destillats S (7), das besteht aus einem wassergesättigten Gemisch von 72 Gew.-% MEK und 18 Gew.-% EtOAc, das teils als Extraktrücklauf S (8) dient und teils zur Befreiung von restlichem Wasser der Kolonne K (4) als S (9) zugeführt wird, und unter Bildung von Wasser S (10) im Sumpf, das zur Extraktiv-Rektifikation in die Kolonne K (1) zurückgeführt wird, so daß sich die Kolonnen K (3) und K (4) betriebsstabil gegen Restkonzentrationen im Strom S (21) verhalten;

n) in einer Kolonne K (4) das etwa 10 Gew.-% Wasser enthaltende MEK/EtOAc-Gemisch durch Azeotrop-Rektifikation mit einem Äther, vorzugsweise MTBE, entwässert wird, wobei das im Verhältnis 4:1 anfallende, wasserfreie Mischlösungsmittel S (22) aus MEK und EtOAc dampfförmig aus dem Sumpf abgezogen wird, um ein saüberes Produkt zu gewährleisten;

o) die aus der Gegenstrom-Extraktion stammende, hauptsächlich EtOAc enthaltende, wassergesättigte Raffinatphase S (15) in einer Kolonne K (5) ebenfalls durch Azeotrop-Rektifikation mit einem Äther, vorzgusweise MTBE, entwässert wird;

p) die Destillate S (17) und S (16) aus den Kolonnen K (4) und K (5), die beide Azeotrope von MTBE mit Wasser sind, gemeinsam als Strom S (18) auf einen gemeinsamen Abscheider A (2) geführt werden, und

q) der wasserfreie Sumpfabzug S (23) aus der Kolonne K (5), der etwa 97 Gew.-% EtOAc, etwa 2 Gew.-% Toluol und etwa 1 Gew.-% MEK enthält, durch einfache kontinuierliche oder diskontinuierliche Rektifikation in einer Kolonne K (6) in Toluol S (25) mit etwa 1 Gew.-% Rest-EtOAc und ein Destillat S (24) mit 99 Gew.-% EtOAc und 1 Gew.-% MEK zerlegt wird.

**4.** Verfahren nach Anspruch 3, dadurch gekennzeichnet, daß zur Durchführung der Azeotrop-Rektifikation in den Kolonnen K (4) und K (5) ein Äther aus derGruppe Ethyläther, Ethylvinyläther, Methylpropyläther, Methylpropenyläther, Isopropylvinyläther, Isopropenylethyläther, Ethylpropyläther, Propylvinyläther, Allylvinyläther, Isopropyläther, cis-1-Butenyl-methyläther, tert-Butenyläther oder trans-1-Butenyl-methyläther verwendet wird.

**5.** Verfahren nach Anspruch 3 oder 4, dadurch gekennzeichnet, daß die Azeotrop-Rektifikation in der Kolonne K (4) und die Azeotrop-Rektifikation in der Kolonne K (5) unter Verwendung des gleichen Äthers als Schleppmittel durchgeführt werden.

## Claims

**1.** A process for separating diacetyl-containing mixtures consisting of methylethyl ketone, ethyl acetate, ethanol and water, which optionally also contain toluene, isopropanol, acetic acid and hydrocarbons, in particular n-hexane, by forming methylethyl ketone (with about 20% by weight of ethyl acetate, at most 0.5% by weight of ethanol and at most 0.1% by weight of water), ethyl acetate (with at most 1% by weight of methylethyl ketone, 0.5% by weight of ethanol and at most 0.05% by weight of water) and ethanol (with at most 0.1% by weight of methylethyl ketone, at most 1% by weight of water and at most 1% by weight of ethyl acetate) of solvent quality for re-use, characterised by the following combination of process steps:

a) the acetic acid optionally contained in the starting mixture is eliminated by neutralisation with sodium carbonate;

b) the diacetyl contained in the neutralised starting mixture, which is troublesome due to its colour and smell, is removed by extractive rectification with water and subsequent reaction with alkali metal or alkaline earth metal hydroxides, alkali metal or alkaline earth metal carbonates or oximes or is withdrawn as a lower side stream in concentrated form just above the bottoms being produced during extractive rectification;

c) the distillate being produced in stage (b) during extractive rectification, which should be free of alcohols, is withdrawn as an upper side stream and separated, by countercurrent extraction with recycling of the extract, into a mixed solvent consisting of about 80% by weight of methylethyl ketone and about 20% by weight ethyl acetate (calculated as anhydrous) and into almost anhydrous ethyl acetate;

d) removal of the residual water from the raffinate being produced in stage (c) during countercurrent extraction, by azeotropic dewatering, using ethers or hydrocarbons whose azeotropic boiling points with water are below 70°C;

e) separation of the bottom products obtained in stage (d) by rectification to give virtually pure ethyl acetate and virtually pure toluene;

f) recovery and recycling of the major part of the water acting as solvent for countercurrent extraction from the extract of countercurrent extraction by distillation and return of some of the organic phase from the condensed ternary methylethyl ketone/ethyl acetate/water mixture thereby being produced to the countercurrent extraction procedure;

g) removal of residual water from the ternary methylethyl ketone/ethyl acetate/water mixture, by azeotropic dewatering, using ethers or hydrocarbons whose azeotropic boiling points with water are below 70°C, with the formation of a bottom product consisting of methylethyl ketone and ethyl acetate in the ratio of 80:20 by weight.

2. A process according to Claim 1, characterised in that

a) removal of residual water from the raffinate from countercurrent extraction in stage (d) of Claim 1 is performed by azeotropic dewatering using ethers or hydrocarbons (preferably using cyclohexane, n-hexane and in particular methyl-tert.-butyl ether), whereby their azeotropic boiling points with water should be below 70°C;

b) recycling of the ternary methylethyl ketone/ethyl acetate/water mixture in accordance with stage (f) in Claim 1 is performed in an extraction stage at the end of the extraction procedure;

c) the inflow (feed) within the extraction procedure (for example in the third stage of a total of 11 stages) takes place between the end of extraction and the start of extraction;

d) removal of residual water from the ternary methylethyl ketone/ethyl acetate/water mixture in accordance with stage (g) of Claim 1 is performed by azeotropic dewatering using ethers or hydrocarbons (preferably using cyclohexane, n-hexane and in particular methyl-tert.-butyl ether) whose azeotropic boiling points with water should be below 70°C, whereby the same entraining agent (ether, hydrocarbon) is preferably used for removing residual water from the raffinate [ethyl acetate/(toluene)/water] and from the crude dewatered extract [ternary methylethyl ketone/ethyl acetate/water mixture];

e) the aqueous phase from the dewatering procedure described in stage (d) above is supplied to water recovery (column 3) and from there, with the distilled distillate (S7) [ternary methylethyl ketone/ethyl acetate/water mixture], again reaches the residual dewatering described in stage (d) above without loss of entraining agent; and

f) after reaction of diacetyl in the remaining aqueous solution of alcohols, preferably ethanol and isopropanol, these alcohols are recovered by distillation.

3. A process according to Claim 1 or 2, characterised in that to separate a starting mixture (feed) consisting of

about 25 to 30% by weight of methylethyl ketone (MEK)

about 25 to 35% by weight of ethyl acetate (EtOAc)

about 20 to 25% by weight of ethanol (EtOH)

about 10 to 15% by weight of water ($H_2O$)

about 1% by weight of isopropanol (IPA)

about 0.5% by weight of toluene (Tol)

about 0.2% by weight of hydrocarbons, preferably n-hexane (Hex)

24

about 2.5% by weight of ethoxypropanol and propylene glycol methyl ether (Etoxy)
about 0.2% by weight of acetic acid (AcOH) and
about 0.2% by weight of diacetyl (Diac)
into re-usable methylethyl ketone (with about 20% by weight of EtOAc, at most 0.5% by weight of EtOH and at most 0.1% by weight of $H_2O$), ethyl acetate (with at most 1% by weight of MEK, at most 0.5% by weight of EtOH and at most 0.05% by weight of $H_2O$) and ethanol (with at most 1% by weight of MEK, at most 1% by weight of $H_2O$ and at most 1% by weight of EtOAc) of solvent quality

a) the starting mixture (feed) is neutralised by the addition of sodium carbonate to eliminate the acetic acid contained therein,

b) the neutralised inflow feed S (1) is separated into the three streams S (2), S (3) and S (5) by extractive rectification in a rectification column K (1) using a recirculated water stream S(4),

c) a mixture S (3) consisting of about 1/4 MEK and about 3/4 n-hexane (with about 1% by weight of water) is withdrawn at the head of column K (1) as the upper phase in a phase separator A (1), while the lower aqueous phase flows back into the column, and the subsequently dried solvent mixture of n-hexane and MEK is taken to another application,

d) with the bottoms S (2) from column K (1) all of the diacetyl together with EtOH, IPA, Etoxy (and any small quantities of sodium acetate from the acetic acid neutralisation stage (a) which are present) is withdrawn in aqueous solution with extensive removal of MEK and EtOAc from the bottoms outflow or

e) the diacetyl, which has been concentrated just above the bottom of K (1), is withdrawn as a lower side stream in concentrated form,

f) a distillate S (5) is withdrawn as an upper side stream from column K (1), which is free of diacetyl and also free of alcohols, consisting of up to about a half of each of MEK and EtOAc along with 5% by weight of water and about 1% by weight of toluene,

g) the rectification auxiliary agent used is water S (4) which is recovered (part of the bottoms withdrawal) from dewatering columns K (2) and K (3) which are described below, whereby small residual amounts of MEK and EtOAc do not cause a problem,

h) the auxiliary agent water for recirculation is recovered in column K (2) from the bottoms product S (2) from column K (1), by withdrawing a mixture of EtOH/IPA/Etoxy/diacetyl/water as distillate S (11), whereby the bottoms stream S (12) is taken to extractive rectification in column K (1) and acts as solvent S (27) or S (14) in the counter-current extraction described below and an amount S (26) of stream S (27) corresponding to the amount of water in the feed and the fresh water influx to solvent S (14) is taken out of the system, which also eliminates the sodium acetate which is formed in small quantities during neutralisation, if it has not already been removed beforehand from the inflow to column K (1),

i) the distillate S (11) is freed of water by producing absolute alcohol in a manner known per se, whereby at the same time the diacetyl is destroyed by boiling by adding small amounts of alkali metal hydroxide or alkali metal carbonate or is chemically bonded by reagents such as hydroxylamine, alkali metal sulphite or the like, which removes diacetyl without loss of EtOAc by saponification or of MEK by reaction, which have both already been removed from the starting mixture, the mixture being withdrawn by removing from the system the waste water arising in very small amounts as a bottom product with the very dark coloured decomposition products from diacetyl,

j) distillate S (5) is fed to the said countercurrent extraction with extract reflux S (8), using water S (14) as solvent,

k) an aqueous solution S (6) is obtained during countercurrent extraction with extract reflux, which contains about 83% by weight of water and which contains MEK and EtOAc in the ratio of at least 4:1, whereas a raffinate S (15) is obtained which consists of a mixture of about 93% by weight of EtOAc and about 3% by weight of water and virtually all the toluene (about 2% by weight),

l) the amounts of extraction agent water and extract reflux are mutually arranged in such a way that the MEK content of the raffinate does not exceed 1% by weight, in order to satisfy the purity requirements of the EtOAc,

m) the extract stream S (6) and the aqueous phase S (21) from separator A (2) are worked up in the pre-dewatering column K (3) with the formation of a distillate S (7), which consists of a mixture, saturated with water, consisting of 72% by weight of MEK and 18% by weight of EtOAc, some of which acts as extract reflux S (8) and some of which is used as S (9) to remove residual water from column K (4), and with the formation of water S (10) in the bottoms which is returned to extractive rectification in column K (1), so that columns K (3) and K (4) behave in an operationally stable manner towards residue concentrations in stream S (21),

n) the MEK/EtOAc mixture which contains about 10% by weight of water is dewatered in column K (4) by azeotropic rectification with an ether, preferably MTBE, whereby the anhydrous mixed solvent S (22) being produced, consisting of MEK and EtOAc in the ratio of 4:1, is withdrawn from the bottoms in the form of vapour, in order to ensure a clean product,

o) the raffinate phase S (15) which arises from countercurrent extraction, containing mainly EtOAc and saturated with water, is dewatered in column K (5), also by azeotropic rectification with an ether, preferably MTBE,

p) distillates S (17) and S (16) from columns K (4) and K (5), which are both azeotropes of MTBE and water, are taken together as stream S (18) to a common separator A (2), and

q) the anhydrous bottoms outflow S (23) from column K (5), which contains about 97% by weight of EtOAc, about 2% by weight of toluene and about 1% by weight of MEK, is separated into toluene S (25) with about 1% by weight of residual EtOAc and a distillate S (24) with 99% EtOAc and 1% by weight of MEK, by simple continuous or batchwise rectification in column K (6).

4. A process according to Claim 3, characterised in that an ether from the group ethyl ether, ethyl vinyl ether, methyl propyl ether, methyl propenyl ether, isopropyl vinyl ether, isopropenyl ethyl ether, ethyl propyl ether, propyl vinyl ether, allyl vinyl ether, isopropyl ether, cis-1-butenyl methyl ether, tert.butenyl ether or trans-1-butenyl methyl ether is used to perform the azeotropic rectification in columns K (4) and K (5).

5. A process according to Claim 3 or 4, characterised in that the azeoptropic rectification in column K (4) and the azeotropic rectification in column K (5) are performed using the same ether as entraining agent.

**Revendications**

1. Procédé de séparation de mélanges de méthyléthylcétone, d'acétate d'éthyle, d'éthanol et d'eau contenant du diacétyle, qui contiennent le cas échéant du toluène, de l'isopropanol, de l'acide acétique et des hydrocarbures, en particulier du n-hexane, avec formation de méthyléthylcétone (avec environ 20 % en poids d'acétate d'éthyle, au maximum 0,5 % en poids d'éthanol et au maximum 0,1 % en poids d'eau), d'acétate d'éthyle (contenant au maximum 1 % en poids de méthyléthylcétone, au maximum 0,5 % en poids d'éthanol et au maximum 0,05 % en poids d'eau) et d'éthanol (contenant au maximum 0,1% en poids de méthyléthylcétone, au maximum 1 % en poids d'eau et au maximum 1 % en poids d'acétate d'éthyle) de qualité pour solvants en vue de la réutilisation, caractérisé par la combinaison de dispositions opératoires suivantes ;

a) l'acide acétique contenu le cas échéant dans le mélange de départ est éliminé par neutralisation avec du carbonate de sodium ;

b) le diacétyle nuisible pour la couleur et pour l'odeur, contenu dans le mélange de départ neutralisé, est éliminé par rectification extractive avec de l'eau puis réaction avec des hydroxydes de métaux alcalins ou alcalino-terreux, des carbonates de métaux alcalins ou alcalino-terreux ou des oximes, ou soutiré comme courant latéral inférieur, sous forme concentrée, à peu de distance au-dessus du résidu de distillation se formant lors de la rectification extractive ;

c) le distillat formé dans le stade b) lors de la rectification extractive, qui doit être exempt d'alcools, est éliminé comme courant latéral supérieur et décomposé par extraction à contre-courant avec renvoi de l'extrait dans un solvant mixte constitué d'environ 80 % en poids de méthyléthylcétone et d'environ 20 % en poids d'acétate d'éthyle (calculés sur la base des poids anhydres) et en acétate d'éthyle presque anhydre ;

d) l'élimination de l'eau résiduelle du raffinat formé dans le stade c) lors de l'extraction à contre-courant, s'effectue par déshydratation azéotropique au moyen d'éthers ou d'hydrocarbures dont les azéotropes avec l'eau ont un point d'ébullition inférieur à 70 °C ;

e) séparation du résidu de distillation obtenu au stade d) par rectification dans de l'acétate d'éthyle pratiquement pur et du toluène pratiquement pur ;

f) récupération et renvoi de la plus grande partie de l'eau servant de solvant pour l'extraction à contre-courant dans l'extrait de l'extraction à contre-courant par distillation et renvoi d'une partie de la phase organique du mélange ternaire méthyléthylcétone-acétate d'éthyle-eau condensé se formant pendant cette opération dans l'extraction à contre-courant ; et

g) élimination de l'eau résiduelle du mélange ternaire méthyléthylcétone-acétate d'éthyle-eau par élimination azéotropique au moyen d'éthers ou d'hydrocarbures dont les azéotropes avec l'eau ont un point d'ébullition inférieur à 70 °C, avec formation d'un résidu de distillation constitué de méthyléthylcétone et d'acétate d'éthyle dans le rapport pondéral 80:20.

2. Procédé selon la revendication 1, caractérisé en ce que :

a) l'élimination de l'eau résiduelle du raffinat de l'extraction à contre-courant dans le stade d) de la revendication 1) est effectué par déshydratation azéotropique au moyen d'éthers ou d'hydrocarbures (de préférence en utilisant du cyclohexane, du n-hexane et en particulier de l'éther méthylique-tert-butylique), les points de fusion de leurs azéotropes avec l'eau devant être inférieurs à 70 °C ;

b) le renvoi du mélange ternaire méthyléthylcétone-acétate d'éthyle-eau selon le stade f) de la revendication 1 à un stade d'extraction s'effectue à la fin de l'extraction ;

c) l'alimentation (feed) à l'intérieur de l'extraction (par exemple au troisième stade sur 11 stades au total) s'effectue entre la fin de l'extraction et le début de l'extraction ;

d) l'élimination de l'eau résiduelle du mélange ternaire méthyléthylcétone-acétate d'éthyle-eau conformément au stade g) de la revendication 1 est effectuée par déshydratation azéotropique au moyen d'éthers ou d'hydrocarbures (de préférence en utilisant du cyclohexane, du n-hexane et en particulier de l'éther méthylique-tert-butylique), dont les azéotropes avec l'eau doivent avoir des points d'ébullition inférieurs à 70 °C, en utilisant de préférence pour l'élimination de l'eau résiduelle du raffinat [acétate d'éthyle/(toluène)/eau] et de l'extrait déshydraté brut [mélange ternaire méthyléthylcétone-acétate d'éthyleeau], le même entraîneur [éthers, hydrocarbures] ;

e) la phase aqueuse de la déshydratation décrite au stade d) ci-dessus est envoyée à la récupération d'eau (colonne 3) et repasse de celle-ci avec le distillat séparé par distillation (S7) [mélange ternaire méthyléthylcétone-acétate d'éthyle-eau] sans entraîneur dans l'élimination de l'eau résiduelle décrite au stade d) ci-dessus ; et

f) après réaction du diacétyle dans la solution aqueuse restante des alcools, de préférence l'éthanol et l'isopropanol, ces alcools sont récupérés par distillation.

3. Procédé selon les revendications 1 ou 2, caractérisé en ce que pour la séparation d'un mélange de départ (feed) constitué

d'environ 25 à 30 % en poids de méthyléthylcétone (MEK),

d'environ 25 à 35 % en poids d'acétate d'éthyle (EtOAc),

d'environ 20 à 25 % en poids d'éthanol (EtOH),

d'environ 10 à 15 % en poids d'eau ($H_2O$),

d'environ 1 % en poids d'isopropanol (IPA),

d'environ 0,5 % en poids de toluène (Tol),

d'environ 0,2 % en poids d'hydrocarbures, de préférence de n-hexane (Hex),

d'environ 2,5 % en poids d'éthoxypropanol et d'éther méthylique du propylène-glycol (Etoxy),

d'environ 0,2 % en poids d'acide acétique (AcOH) et

d'environ 0,2 % en poids de diacétyle (Diac) en méthyléthylcétone réutilisable (contenant environ 20 % en poids d'EtOAc, 0,5 % en poids au maximum d'EtOH et 0,1 % en poids au maximum de $H_2O$), en acétate d'éthyle (contenant 1 % en poids au maximum de MEK, 0,5 % en poids au maximum d'EtOH et 0,05 % en poids au maximum de $H_2O$) et en éthanol (contenant environ 1 % en poids au maximum de MEK, 1 % en poids au maximum de $H_2O$ et 1 % en poids au maximum d'EtOAc) de qualité pour solvants

a) le mélange de départ (feed) est neutralisé par addition de carbonate de sodium pour l'élimination de l'acide acétique qu'il contient ;

b) l'alimentation neutralisée feed S (1) est divisée par rectification extractive dans une colonne de rectification K (1) avec un courant d'eau ramené S (4) en les trois courants S (2), S (3) et S (5) ;

c) en tête de la colonne K (1), on prélève un mélange S (3) d'environ 1/4 de MEK et environ 3/4 de n-hexane, (avec environ 1 % en poids d'eau) comme phase supérieure dans un séparateur de phases A (1), la phase aqueuse inférieure revenant dans la colonne et le mélange de solvants de n-hexane de MEK séché étant renvoyé à l'utilisation ultérieure ;

d) avec le résidu de distillation S (2) de la colonne K (1), la totalité du diacétyle est soutirée en même temps que EtOH, IPA, Etoxy ainsi que de faibles quantités éventuellement présentes d'acétate de sodium provenant du stade de neutralisation de l'acide acétique a) en solution aqueuse, avec élimination poussée de la MEK et du EtOAc de l'écoulement du résidu de distillation ou

e) le diacétyle, qui s'est enrichi à peu de distance au-dessus du résidu de distillation de K (1), est soutiré comme courant latéral inférieur sous forme concentrée ;

f) comme courant latéral supérieur provenant de la colonne K (1), on soutire un distillat S (5) qui est exempt de diacétyle et également d'alcools et se compose à peu près pour moitié de MEK et de EtOAc, avec en outre 5 % en poids d'eau et environ 1 % en poids de toluène ;

g) comme adjuvant de rectification, on utilise de l'eau S (4), qui est un produit de régénération (une partie du soutirage du résidu de distillation) provenant des colonnes de déshydratation K (2) et K (3) décrites plus loin ci-dessous, de faibles teneurs résiduelles en MEK et EtAOc ne gênant pas ;

h) dans une colonne K (2), à partir du produit de distillation S (2) de la colonne K (1), on récupère l'eau servant d'auxiliaire pour les cycles, en soutirant comme distillat S (11) un mélange EtOH/IPA/Etoxy/diacétyle/eau, le courant de résidu de distillation S (12) servant pour la rectification extractive dans la colonne K (1) et comme solvant S (27) ou S (14) pour l'extraction à contre-courant décrite ci-dessous, et une quantité S (26) du courant S (27) correspondant à l'eau contenue dans le feed et à l'apport d'eau fraîche dans le solvant S (14) étant éclusés, grâce à quoi même l'acétate de sodium formé en faibles quantités lors de la neutralisation est éliminé, au cas où il ne l'a pas déjà été auparavant de l'alimentation de la colonne K (1) ;

i) le distillat S (11) est débarrassé de son eau d'une manière connue en soi par "absolutisation" par l'alcool, le diacétyle étant simultanément détruit par addition de petites quantités d'hydroxyde alcalin ou de carbonate alcalin par ébullition, ou lié chimiquement par des réactifs tels que l'hydroxylamine, un sulfite alcalin, etc., le diacétyle étant éliminé sans pertes du mélange par saponification de l'EtOAc ou par réaction de la MEK, qui ont déjà été tous les deux éliminés du mélange de départ, par évacuation de l'eau résiduaire se formant en très faible quantité avec des produits de décomposition d'u noir interne du diacétyle comme résidu de distillation ;

j) le distillat S (5) est envoyé à l'extraction à contre-courant effectuée avec l'eau S (14) comme solvant avec le reflux de l'extrait S (8) ;

k) dans l'extraction à contre-courant avec reflux de l'extrait on obtient comme extrait une solution aqueuse S (6) qui contient environ 83 % en poids d'eau et qui contient la MEK et l'EtOAc dans un rapport d'au moins 4:1, tandis que l'on obtient comme raffinat S (15) un mélange d'environ 93 % en poids d'EtOAc et d'environ 3 % en poids d'eau, ainsi que de la quasi-totalité du toluène d'environ 2 % en poids) ;

l) les quantités de d'eau utilisée comme agent d'extraction et de reflux de l'extrait doivent être accordées l'une à l'autre de telle manière que la teneur en MEK du raffinat 1 ne dépasse pas 1 % en poids pour satisfaire aux exigences de pureté de l'EtOAc ;

m) le courant d'extrait S (6) et la phase aqueuse S (21) du séparateur A (2) sont traités dans la colonne de prédéshydratation K (3) avec formation d'un distillat S (7), qui se compose d'un mélange saturé d'eau de 72 % en poids de MEK et de 18 % en poids d'EtAOc, qui sert pour une part comme reflux d'extrait S (8) et est envoyé pour une autre part pour l'élimination de l'eau résiduaire de la colonne K (4), en tant que S (9), et avec formation d'eau S (10) dans le résidu de distillation qui est renvoyé à la rectification extractive dans la colonne K (1), de telle sorte que les colonnes K (3) et K (4) ont un comportement stable vis-à-vis des concentrations résiduelles dans le courant S (21) ;

n) dans une colonne K (4), le mélange MEK/EtOAc contenant environ 10 % en poids d'eau est déshydraté par rectification azéotropique avec un éther, de préférence le MTBE, le solvant mixte exempt d'eau S (22) constitué de MEK et EtOAc se formant dans le rapport 4:1 est éliminé sous forme de vapeurs du résidu de distillation pour garantir un produit propre ;

o) la phase raffinat S (15) saturée d'eau provenant de l'extraction à contre-courant, contenant principalement EtOAc, est déshydratée dans une colonne K (5), également par rectification azéotropique avec un éther, de préférence le MBTE ;

p) les distillats S (17) et S (16) des colonnes K (4) et K (5), qui sont tous les deux des azéotropes de MTBE avec l'eau, sont envoyés en commun comme courants S (18) à un séparateur commun A (2), et

q) le soutirage du résidu de distillation exempt d'eau S (23) de la colonne K (5), qui contient environ 97 % en poids d'EtOAc, environ 2 % en poids de toluène et environ 1 % en poids de MEK, est décomposé par une simple rectification continue ou discontinue dans une colonne K (6) en toluène S (25) avec environ 1 % en poids d'EtOAc résiduel et en un distillat S (24) contenant 99 % en poids d'EtOAc et 1 % en poids de MEK.

4. Procédé selon la revendication 3, caractérisé en ce que pour effectuer la rectification azéotropique dans les colonnes K (4) et K (5), on utilise un éther choisi parmi l'éther éthylique, l'éther éthylique vinylique, l'éther méthylique propylique, l'éther méthylique propénylique, l'éther isopropylique vinylique, l'éther isopropénylique éthylique, l'éther éthylique propylique, l'éther propylique vinylique, l'éther allylique vinylique, l'éther isopropylique, l'éther cis-1-buténylique-méthylique, l'éther tert-buténylique ou de l'éther trans-1-buténylique.

5. Procédé selon les revendications 3 ou 4, caractérisé en ce que la rectification azéotropique dans la colonne K (4) et la rectification azéotropique dans la colonne K (5) sont effectuées en utilisant le même éther comme entraîneur.

Hexan

Feed

S(1)

S(5)

S(3)

S(6)

S(8)

S(15)

⇐⊐ (Frischwasser)

S(17)

⇐⊐ (MTBE)

Extraktion

K1

K2

K3

K4

K5

K6

S(4)

S(7)

S(9)

S(14)

A2

S(18)

S(16)

S(24)

EtOAc

S(19)  S(20)

S(12)

S(27)

S(21)

S(2)

S(13)

S(10)

S(26)

S(22)

S(23)

S(25)

EtOH
Wasser
Ethoxy
DiAc
IPA

S(11)

Wasser-
ausschleusung

MEK
EtOAc

Toluol

EP 0 384 458 B1

30

Abb. 1   K1: Extraktivrektifikation mit Wasser
K2: Wasserrückgewinnung
K3: Lösungsmittelrückgewinnung für die Extraktion
K4: Extraktentwässerung
K5: Raffinatentwässerung
K6: Toluolabtrennung

Abb.2   K1: Extraktivrektifikation mit Wasser
        K2: Wasserrückgewinnung
        K3: Lösungsmittelrückgewinnung für die Extraktion
        K4: Extraktentwässerung
        K5: Raffinatentwässerung
        K6: Toluolabtrennung

435 kg/h MEK
465 kg/h EtOAc
7.6 kg/h Tol
192 kg/h H$_2$O
7.6 kg/h Hex

337 kg/h EtOH
15 kg/h IPA
37 kg/h Ethoxy
3 kg/h DiAc

10644 kg/h H$_2$O
337 kg/h EtOH
37 kg/h Ethoxy
15 kg/h IPA
3 kg/h DiAc

S(1)

433 kg/h MEK
465 kg/h EtOAc
48 kg/h H$_2$O
7.6 kg/h Tol

K1 Extraktivrektification mit Wasser (v=2.7)

7.6 kg/h Hex
2.2 kg/h MEK
0.1 kg/h H$_2$O

S(3)

S(2)

S(4)

10500 kg/h H$_2$O

S(5)

4082 kg/h H$_2$O

337 kg/h EtOH
15 kg/h IPA
37 kg/h Ethoxy
3 kg/h DiAc
54 kg/h H$_2$O

S(11)

K2 Wasserrückgewinnung (v=1.5)

S(12)

6508 kg/h H$_2$O

S(27)

S(13)

S(26)

138 kg/h H$_2$O

(Frischwasser)

S(14)    6370 kg/h H$_2$O

6496 kg/h H$_2$O
1073 kg/h MEK
268 kg/h EtOAc

Extraktion

S(8)

644 Kg/h MEK
161 kg/h EtOAc
90 kg/h H$_2$O
0.8 kg/h MTBE

S(6)

S(10)

S(15)

358 kg/h EtOAc
12 kg/h H$_2$O
7.6 kg/h Tol
4 kg/h MEK
2.5 kg/h MTBE

K3 Lösungsmittelrückgewinnung (v=1.5)

6418 kg/h H$_2$O

S(9)

S(21)

429 kg/h MEK
107 kg/h EtOAc
60 kg/h H$_2$O
1.7 kg/h MTBE

72 kg/h H$_2$O
4.2 kg/h MTBE

K5 Raffinatentwässerung

S(20)    S(19)

K4 Extraktentwässerung

1253 kg/h MTBE
32 kg/h H$_2$O

1251 kg/h MTBE
20 kg/h H$_2$O

3539 kg/h MTBE
55 kg/h H$_2$O

S(22)

429 kg/h MEK
107 kg/h EtOAc

S(16)

Abscheider

S(17)

358 kg/h EtOAc
7.6 kg/h Toluol
4 kg/h MEK

S(23)

3541 kg/h MTBE
115 kg/h H$_2$O

Abb.3

K6 Toluolabtrennung (v=0.5)

S(25)

7.6 kg/h Toluol
7.6 kg/h EtOAc

S(24)

350 kg/h EtOAc
4 kg/h MEK

Wasser
EtOAc
MEK
Ethanol
MTBE
Rückläufe